# EUROPEAN PATENT APPLICATION

(11) **EP 3 428 272 A1**
(43) Date of publication of application: **16.01.2019**
(21) Application number: 17763395.5
(22) Date of filing: 09.03.2017
(51) Int. Cl.: C12N 5/071, C12N 5/0775, C12M 1/00

(54) **METHOD FOR MANUFACTURING CULTURE PRODUCT LIQUID**

(30) Priority: 10.03.2016 JP 2016047119
(71) Applicant: KintaroCellsPower Co., Ltd., Shinagawa-ku Tokyo 1410022 (JP)
(72) Inventor: GLADKOV, Alexei, Tokyo 108-0074 (JP)
(74) Representative: Ashton, Gareth Mark
(86) International application number: PCT/JP2017/009546
(87) International publication number: WO 2017/155055

(57) **Abstract**

[Problem] To provide a method for manufacturing a culture product liquid, whereby a culture product liquid is manufactured which includes a predetermined metabolite secreted from a single type of stem cell.

[Solution] The method for manufacturing a culture product liquid according to the present invention comprises: extracting an intermediate-layer bone marrow liquid positioned in an intermediate layer from bone marrow liquid separated in layered fashion and culturing the intermediate-layer bone marrow liquid together with a culture liquid; fixing first stem cells to a bottom surface of a first culture container and collecting the first stem cells from the first culture container when the total area of the first stem cells reaches a first target ratio of the bottom surface area of the first culture container; culturing second stem cells together with the culture liquid, the second stem cells being positioned in a bottom layer from among the first stem cells separated in layered fashion, while collecting the second stem cells; and fixing the second stem cells to the bottom surface of a second culture container, and extracting a culture product liquid including a predetermined metabolite secreted from a single type of second stem cells grown from the second culture container when the total area of the second stem cells reaches a second target ratio of the bottom-surface area of the second culture container.

## Description

### TECHNICAL FIELD

The present invention relates to a method for manufacturing a culture product liquid that manufactures a culture product liquid, using a stem cell made from a bone marrow liquid collected from a donor.

### BACKGROUND

Conventionally disclosed is a stem cell culture apparatus including a culture tank, a culture liquid filling up inside the culture tank, and a support having a height difference on the support surface and coated with a stem cell-adhesion material, in which a plurality of lower portions providing a height difference on the support surface is present (see Patent Document 1). The stem cell culture apparatus is configured to float the support in the culture liquid, and proliferate the stem cell, which is adhered on the support surface through the stem cell adhesion material in a dispersed condition, without peeling-off from the support surface.

### PRIOR ART DOCUMENTS

### PATENT DOCUMENTS

Patent Document 1: JP-A-2014-183770

### SUMMARY OF THE INVENTION

### PROBLEM TO BE SOLVED BY THE INVENTION

In the stem cell culture apparatus disclosed in Patent Document 1, a culture liquid including cultured stem cells is discarded as waste material. The culture liquid including the cultured stem cells, containing metabolites secreted from these stem cells, can be used for disease treatment and cosmetic uses. Nevertheless, since various types of stem cells are present in a tissue or its cells collected from a donor, culturing such stem cells leads to their own proliferation, and the resulting various types of metabolites secreted from these stem cells are contained in the culture liquid, thereby unsuccessfully manufacturing a culture product liquid having the only specific effect on each disease treatment or cosmetic use.

The present invention has an object to provide a method for manufacturing a culture product liquid capable of manufacturing a culture product liquid containing a predetermined metabolite secreted from a single type of stem cell and having the only specific effect on each disease treatment or cosmetic use.

### MEANS FOR SOLVING THE PROBLEM

To solve these problems, the present invention provides a method for manufacturing a culture product liquid that manufactures a culture product liquid, using a stem cell made from a bone marrow liquid collected from a donor.

The method for manufacturing a culture product liquid according to the present invention includes: separating a bone marrow liquid for separating in layers a bone marrow liquid collected from a donor; extracting a bone marrow liquid for extracting an intermediate-layer bone marrow liquid positioned in an intermediate layer of the bone marrow liquid separated in layers by the step of separating a bone marrow liquid; first fixing a stem cell for feeding the intermediate-layer bone marrow liquid extracted by the step of extracting a bone marrow liquid and a predetermined culture liquid into a first culture container having a predetermined capacity and a bottom surface of a predetermined area, allowing the first culture container to statically stand for a predetermined period of time, and fixing a first stem cell contained in the intermediate-layer bone marrow liquid to a bottom surface of the first culture container; first culturing a stem cell for discharging the culture liquid in the first culture container after fixing the first stem cell to the bottom surface of the first culture container by the step of first fixing a stem cell, feeding a new culture liquid into the first culture container, allowing the first culture container to statically stand for a predetermined period of time, and culturing the first stem cell until the total area of the first stem cell reaches a first target ratio of the bottom-surface area of the first culture container; first collecting a stem cell for collecting the first stem cell from the first culture container when the total area of the first stem cell reaches the first target ratio in the process of culturing the first stem cell by the step of first culturing a stem cell; separating a stem cell for centrifugally separating in layers the first stem cell collected by the step of first collecting a stem cell; second collecting a stem cell for collecting a second stem cell positioned in the lowermost layer of the first stem cell separated in layers by the step of separating a stem cell; second fixing a stem cell for feeding the second stem cell collected by the step of second collecting a stem cell and a predetermined culture liquid into a second culture container having a larger capacity and a bottom surface of a larger bottom-surface area than the first culture container, allowing the second culture container to statically stand for a predetermined period of time, and fixing the second stem cell to a bottom surface of the second culture container; second culturing a stem cell for discharging the culture liquid in the second culture container after fixing the second stem cell to the bottom surface of the second culture container by the step of second fixing a stem cell, feeding a new culture liquid into the second culture container, allowing the second culture container to statically stand for a predetermined period of time, and culturing the second stem cell until the total area of the second stem cell reaches a second target ratio of the bottom-surface area of the second culture container; and first extracting a culture product liquid for extracting from the second culture container a culture product liquid containing a predetermined metabolite secreted from the single type of second stem cell proliferated when the total area of the second stem cell reaches the second target ratio in the process of culturing in the step of second culturing a stem cell.

As one example of the method for manufacturing a culture product liquid of the present invention, in the steps of first fixing a stem cell and first culturing a stem cell, the first culture container is allowed to statically stand at a predetermined tilted angle for a predetermined period of time, and in the steps of second fixing a stem cell and second culturing a stem cell, the second culture container is allowed to statically stand at a predetermined tilted angle for a predetermined period of time.

As another example of the method for manufacturing a culture product liquid of the present invention, the method for manufacturing a culture product liquid includes: second collecting a stem cell for collecting the second stem cell from the second culture container after extracting a culture product liquid from the second culture container by the step of first extracting a culture product liquid; third fixing a stem cell for feeding the second stem cell collected by second collecting a stem cell and a new culture liquid into a third culture container having a predetermined capacity and a bottom surface of a predetermined area, the capacity and the bottom-surface area being larger than the second culture container, allowing the third culture container to statically stand for a predetermined period of time, and fixing the second stem cell to a bottom surface of the third culture container; third culturing a stem cell for discharging the culture liquid in the third culture container after fixing the second stem cell to the bottom surface of the third culture by the step of third fixing a stem cell, feeding a new culture liquid into the third culture container, allowing the third culture container to statically stand for a predetermined period of time, and culturing the second stem cell until the total area of the second stem cell reaches a third target ratio of the bottom-surface area of the third culture container; and second extracting a culture product liquid for extracting from the third culture container a culture product liquid containing a predetermined metabolite secreted from the single type of second stem cell proliferated when the total area of the second stem cell reaches the third target ratio in the process of culturing in the step of third culturing a stem cell.

As another example of the method for manufacturing a culture product liquid of the present invention, in the steps of third fixing a stem cell and third culturing a stem cell, the third culture container is allowed to statically stand at a predetermined tilted angle for a predetermined period of time.

As another example of the method for manufacturing a culture product liquid of the present invention, in the step of separating a bone marrow liquid, 2 to 3cc of a bone marrow liquid is collected from the donor, 2 to 3cc of the bone marrow liquid is fed into a vertically elongating separation container, the separation container is allowed to statically stand at approximately the same temperature as the body temperature for a predetermined period of time, and the bone marrow liquid is vertically separated in layers in the separation container, and in the step of extracting a bone marrow liquid, and the intermediate-layer bone marrow liquid positioned in the intermediate layer of the bone marrow liquid separated in layers in the separation container is extracted.

As another example of the method for manufacturing a culture product liquid of the present invention, the capacity of the first culture container is approx. 20 to 30cc, the initial plane shape of the first stem cell is an approximately circular shape, and the plane shape of the first stem cell deformed is primarily the approximately circular shape, and a flat shape when the first stem cell elongates in amorphous form in one direction, and in the step of first fixing a stem cell, the first culture container is allowed to statically stand at approximately the same temperature as the body temperature for a period of 12 to 24 hours, the deformation of the first stem cell from the initial plane shape in the first culture container is observed at an interval of approx. 1 to 2 hours for the period of 12 to 24 hours, and when the first stem cell is deformed from the initial plane shape to the amorphous flat shape, it is determined that the first stem cell is fixed to the bottom surface of the first culture container.

As another example of the method for manufacturing a culture product liquid of the present invention, in the step of first collecting a stem cell, the first culture container is allowed to statically stand at approximately the same temperature as the body temperature for a period of 36 to 48 hours, and the total area of the first stem cell fixed to the bottom surface of the first culture container in relation to the bottom-surface area of the first culture container area is observed at an interval of approx. 1 to 2 hours for the period of 36 to 48 hours.

As another example of the method for manufacturing a culture product liquid of the present invention, the first target ratio of the total area of the first stem cell is 70 to 80% of the bottom-surface area of the first culture container.

As another example of the method for manufacturing a culture product liquid of the present invention, in the step of separating a stem cell, the first stem cell is fed into a separation container, the separation container is placed in a centrifugal separator to centrifugally separate the first stem cell, and in the step of first collecting a stem cell, the second stem cell positioned in the lowermost layer of the first stem cell centrifugally separated in layers in the separation container is collected.

As another example of the method for manufacturing a culture product liquid of the present invention, the capacity of the second culture container is approx. 40 to 60cc, the capacity of the third culture container is approx. 70 to 80cc, the initial plane shape of the second stem cell is an approximately circular shape, the plane shape of the second stem cell deformed is primarily the approximately circular shape, and a flat shape when the second stem cell elongates in amorphous form in one direction, in the steps of second fixing a stem cell and third fixing a stem cell, the second and the third culture containers are allowed to statically stand at approximately the same temperature as the body temperature for a period of 36 to 48 hours, the deformation of the second stem cell from the initial plane shape in the second and third culture containers is observed at an interval of approx. 1 to 2 hours for a period of 36 to 48 hours, and when the second stem cell is deformed from the initial plane shape to the amorphous flat shape, it is determined that the second stem cell is fixed to the bottom surface of the second and the third culture containers.

As another example of the method for manufacturing a culture product liquid of the present invention, in the steps of second collecting the stem cell and third collecting the stem cell, the second and the third culture containers are allowed to statically stand at approximately the same temperature as the body temperature for a period of 36 to 48 hours, the total area of the second stem cell fixed to the bottom surface of the second and the third culture containers in relation to the bottom-surface area of the second and the third culture containers is observed at an interval of approx. 1 to 2 hours for the period of 36 to 48 hours.

As another example of the method for manufacturing a culture product liquid of the present invention, the second target ratio of the total area of the second stem cell is 88 to 92% of the bottom-surface area of the second culture container, and the third target ratio of the total area of the third stem cell is 88 to 92% of the bottom-surface area of the third culture container.

As another example of the method for manufacturing a culture product liquid of the present invention, the first and second stem cells are mesenchymal stem cells.

### EFFECT OF THE INVENTION

The method for manufacturing a culture product liquid according to the present invention extracts an intermediate-layer bone marrow liquid positioned in an intermediate layer of the bone marrow liquid separated in layers, cultures the intermediate-layer bone marrow liquid together with a culture liquid, fixes a first stem cell to a bottom surface of the first culture container, collects the first stem cell from the first culture container when the total area of the first stem cell reaches a first target ratio of the bottom-surface area of the first culture container area while culturing the first stem cell, collects a second stem cell positioned in the lowermost layer of the first stem cell separated in layers, cultures the second stem cell together with a culture liquid, fixes the second stem cell to a bottom surface of a second culture container, feeds a new culture liquid into the second culture container, cultures the second stem cell, and extracts from the second culture container a culture product liquid containing a predetermined metabolite secreted from the single type of second stem cell proliferated when the total area of the second stem cell reaches a second target ratio of the bottom-surface area of the second culture container in the process of culturing the second stem cell, therefore using the single type of second stem cell cultured, various types of metabolites secreted from various types of stem cells are not contained, but only a predetermined metabolite secreted from the single type of second stem cell is contained, and a culture product liquid capable of providing the only specific effect on predetermined diseases, and predetermined cosmetic uses such as skin care, hair care, and body care can be manufactured, a culture product liquid capable of effective use in treating predetermined diseases can be manufactured, and a culture product liquid capable of effective use in predetermined cosmetic uses can be manufactured. The method for manufacturing a culture product liquid can manufacture a culture product liquid, using a pure (clear) second stem cell obtained by removing unnecessary stem cell, and allow the culture product liquid to contain only a predetermined metabolite secreted from a single type of second stem cell, thereby providing a significant effect of treating predetermined diseases and manufacturing a culture product liquid effective in curing such diseases, which makes it possible to manufacture a culture product liquid having a significant effect on predetermined cosmetic uses.

The method for manufacturing a culture product liquid, which allows the first culture container to statically stand at a predetermined tilted angle for a predetermined period of time in the steps of first fixing a stem cell and first culturing a stem cell, and allows the second culture container to statically stand at a predetermined tilted angle for a predetermined period of time in the steps of second fixing a stem cell and second culturing a stem cell, can assuredly fix the first stem cell to the bottom surface of the first culture container and assuredly encourage the proliferation of the first stem cell by allowing the first culture container to statically stand at a predetermined tilted angle for a predetermined period of time in the steps of first fixing a stem cell and first culturing a stem cell. The method for manufacturing a culture product liquid can assuredly fix the second stem cell to the bottom surface of the second culture container and assuredly encourage the proliferation of the second stem cell by allowing the second culture container to statically stand at a predetermined tilted angle for a predetermined period of time in the steps of second fixing a stem cell and second culturing a stem cell.

The method for manufacturing a culture product liquid, which collects the second stem cell from the second culture container after extracting a culture product liquid from the second culture container, cultures the second stem cell together with a culture liquid, fixes the second stem cell to a bottom surface of a third culture container having a larger capacity and a bottom of a larger bottom-surface area than the second culture container, feeds a new culture liquid into the third culture container, cultures the second stem cell, and extracts from the third culture container a culture product liquid containing a predetermined metabolite secreted from a single type of second stem cell proliferated when the total area of the second stem cell reaches the third target ratio of the bottom-surface area of the third culture container in the process of culturing the second stem cell, further cultures the second stem cell in the third culture container, extracts from the third culture container a culture product liquid containing a predetermined metabolite secreted from the single type of second stem cell proliferated when the total area of the second stem cell reaches the third target ratio of the bottom-surface area of the third culture container in the process of culturing the second stem cell, therefore in the process of culturing the stem cell, various types of stem cells are not cultured, various types of metabolites secreted from various types of stem cells are not contained, only a predetermined metabolite secreted from the single type of second stem cell is contained, a culture product liquid providing the only specific effect on predetermined disease and predetermined cosmetic uses can be manufactured, a culture product liquid capable of effective use in treating predetermined diseases can be manufactured, and a culture product liquid capable of effective use in predetermined cosmetic uses can be manufactured. The method for manufacturing a culture product liquid can manufacture a large amount of culture product liquid at one time using a third culture container having a larger capacity than the second culture container.

The method for manufacturing a culture product liquid, which allows the third culture container to statically stand at a predetermined tilted angle for a predetermined period of time in the steps of third fixing a stem cell and third culturing a stem cell, can assuredly fix second stem cell to a bottom surface of the second culture container and assuredly encourage the proliferation of the second stem cell by allowing the third culture container to statically stand at a predetermined tilted angle for a predetermined period of time in the steps of third fixing a stem cell and third culturing a stem cell.

The method for manufacturing a culture product liquid, which collects 2 to 3cc of a bone marrow liquid from a donor by separating a bone marrow liquid, feeds 2 to 3cc of a bone marrow liquid into a vertically elongating separation container, allows the separation container to statically stand at approximately the same temperature as the body temperature for a predetermined period of time, vertically separate the bone marrow liquid in layers in the separation container, and extracts the intermediate-layer bone marrow liquid positioned in the intermediate layer of the bone marrow liquid separated in layers in the separation container in the step of extracting a bone marrow liquid, allows a bone marrow liquid containing various types of stem cells to statically stand at approximately the same temperature as the body temperature for a predetermined period of time to be vertically separated in layers, thereby assuredly extracting a specific intermediate-layer bone marrow liquid from the bone marrow liquid and culturing a single type of second stem cell from the intermediate-layer bone marrow liquid, therefore a culture product liquid containing only a predetermined metabolite secreted from a single type of stem cell obtained by removing unnecessary stem cell and having the only specific effect on predetermined diseases and predetermined cosmetic uses can be manufactured, a culture product liquid capable of effective use in treating predetermined diseases can be manufactured, and a culture product liquid capable of effective use in predetermined cosmetic uses can be manufactured.

The method for manufacturing a culture product liquid, in which the capacity of the first culture container is approx. 20 to 30cc, the initial plane shape of the first stem cell is an approximately circular shape, the plane shape of the first stem cell deformed is primarily the approximately circular shape, and a flat shape when the first stem cell elongates in amorphous form in one direction, and in the step of first fixing stem cell, the first culture container is allowed to statically stand at approximately the same temperature as the body temperature for a period of 12 to 24 hours, the deformation of the first stem cell from the initial plane shape in the first culture container is observed at an interval of approx. 1 to 2 hours for the period of 12 to 24 hours, and when the first stem cell is deformed from the initial plane shape to the amorphous flat shape, it is determined that the first stem cell is fixed to the bottom surface of the first culture container, fails to fix the first stem cell to the bottom surface of the first culture container when the capacity of the first culture container exceeds 30cc and slows down the proliferation of the first stem cell, but immediately and readily fixes the first stem cell to the bottom surface of the first culture container, using the first culture container having the capacity, manufactures a single type of stem cell for a short period of time by swift proliferation of the first stem cell in the first culture container, and can manufacture a large amount of culture product liquid containing only a predetermined metabolite secreted from a single type of stem cell obtained by removing unnecessary stem cell for a short period of time. The method for manufacturing a culture product liquid allows the first culture container to statically stand at approximately the same temperature as the body temperature for a period of 12 to 24 hours, observes the deformation of the first stem cell first from the initial plane shape in the culture container at an interval of approx. 1 to 2 hours for the period of 12 to 24 hours, and determines that the first stem cell is fixed to the bottom surface of the first culture container when the first stem cell is deformed to provide a primarily approximately circular shape, and a flat shape when the first stem cell elongates in amorphous form in one direction, thus never fails to confirm the fixing of the first stem cell to the bottom surface of the first culture container, properly confirms the fixing of the first stem cell to the bottom surface of the first culture container according to changes in the plane shape of the first stem cell, discharges the culture liquid in the first culture container after confirming that the first stem cell is fixed to the bottom surface of the first culture container, feeds a new culture liquid into the first culture container to assuredly encourage the proliferation of the first stem cell, and using the first stem cell, can assuredly manufacture a culture product liquid containing only a predetermined metabolite secreted from a single type of stem cell.

The method for manufacturing a culture product liquid, which allows the first culture container to statically stand at approximately the same temperature as the body temperature for a period of 36 to 48 hours in the step of first collecting a stem cell and observes the total area of the first stem cell fixed to the bottom surface of the first culture container in relation to the bottom-surface area of the first culture container area at an interval of approx. 1 to 2 hours for a period of 36 to 48 hours, can properly confirm the total area of the first stem cell in relation to the bottom-surface area of the first culture container area by observing the total area at an interval of approx. 1 to 2 hours, can assuredly confirm that the total area of the first stem cell reaches a first target ratio of the bottom-surface area of the first culture container area, holds the activity of the first stem cell without failing to collect the first stem cell from the first culture container with good timing, collects the first stem cell from the first culture container, and using the first stem cell, can assuredly manufacture a culture product liquid containing only a predetermined metabolite secreted from a single type of stem cell.

The method for manufacturing a culture product liquid, in which the first target ratio of the total area of the first stem cell is 70 to 80% of the bottom-surface area of the first culture container area, gradually reduces the activity of the first stem cell when the total area of the first stem cell in relation to the bottom-surface area of the first culture container exceeds 80% to proliferate the first stem cell, and collects the first stem cell from the first culture container when the total area of the first stem cell in relation to the bottom-surface area of the first culture container increases to 70 to 80% for proliferation, thereby maintaining the activity of the first stem cell, accordingly proliferating the first stem cell, and assuredly manufacturing a culture product liquid containing only a predetermined metabolite secreted from a single type of stem cell using the first stem cell.

The method for manufacturing a culture product liquid, which feeds the first stem cell into a separation container in the step of separating a stem cell, places the separation container in a centrifugal separator to centrifugally separate the first stem cell, and collects the second stem cell positioned in the lowermost layer of the first stem cell centrifugally separated in layers in the separation container in the step of first collecting a stem cell, centrifugally separates in layers the first stem cell containing unnecessary stem cell and collects the second stem cell positioned in the lowermost layer of the first stem cell centrifugally separated in layers, thereby assuredly extracting a specific second stem cell from the first stem cell and removing unnecessary stem cell from the first stem cell. The method for manufacturing a culture product liquid can culture only a specific type of second stem cell to be cultured, and assuredly manufacture a culture product liquid containing only a predetermined metabolite secreted from a single type of stem cell using the first stem cell.

The method for manufacturing a culture product liquid, in which the capacity of the second culture container is approx. 40 to 60cc, the capacity of the third culture container is approx. 70 to 80cc, the initial plane shape of the second stem cell is an approximately circular shape, the plane shape of the second stem cell deformed is primarily the approximately circular shape, and a flat shape when the second stem cell elongates in amorphous form in one direction, and in the steps of second fixing a stem cell and third fixing a stem cell, the second and the third culture container are allowed to statically stand at approximately the same temperature as the body temperature for 36 to 48 hour, the deformation of the second stem cell from the initial plane shape in the second and third culture container is observed at an interval of approx. 1 to 2 hours for a period of 36 to 48 hours, and when the second stem cell is deformed from the initial plane shape to the amorphous flat shape, it is determined that the second stem cell is fixed to the bottom surface of the second and third culture containers, fails to readily fix the second stem cell to the bottom surface of the second and the third culture containers when the capacity of the second culture container exceeds 60cc and the capacity of the third culture container exceeds 80cc and slows down the proliferation of the second stem cell, but immediately and readily fixes the second stem cell to the bottom surface of the second and the third culture containers, using the second and the third culture containers having the capacities, manufactures a single type of second stem cell for a short period of time by swift proliferation of the second stem cell in the second and the third culture containers, and can manufacture a large amount of culture product liquid containing only a predetermined metabolite secreted from a single type of second stem cell obtained by removing unnecessary stem cell for a short period of time. The method for manufacturing a culture product liquid allows the second and the third culture containers to statically stand at approximately the same temperature as the body temperature for a period of 36 to 48 hours, observes the deformation of the second stem cell from the initial plane shape in the second and the third culture containers at an interval of approx. 1 to 2 hours for a period of 36 to 48 hours, and determines that the second stem cell is fixed to the bottom surface of the second and the third culture containers when the second stem cell is deformed to provide a primarily approximately circular shape, and a flat shape when the second stem cell elongates in amorphous form in one direction, thus never fails to confirm the fixing of the second stem cell to the bottom surface of the second and the third culture containers, properly confirms the fixing of the second stem cell to the bottom surface of the second and the third culture containers according to changes in the plane shape of the second stem cell, discharges the culture liquid in the second and the third culture containers after confirming that the second stem cell is fixed to the bottom surface of the second and the third culture containers, feeds a new culture liquid into the second and the third culture containers to assuredly encourages the proliferation of the second stem cell, and using the second stem cell, can assuredly manufacture a culture product liquid containing only a predetermined metabolite secreted from a single type of second stem cell.

The method for manufacturing a culture product liquid, which allows the second and the third culture containers to statically stand at approximately the same temperature as the body temperature for a period of 36 to 48 hours in the steps of second and third collecting a stem cell and observes the total area of the second stem cell fixed to the bottom surface of the second and the third culture containers in relation to the bottom-surface area of the second and the third culture containers at an interval of approx. 1 to 2 hours for a period of 36 to 48 hours, can properly confirm the total area of the second stem cell in relation to the bottom-surface area of the second and the third culture containers by observing the total area at an interval of approx. 1 to 2 hours, can assuredly confirm that the total area of the second stem cell reaches second and third target ratios of the bottom-surface areas of the second and the third culture containers, holds the activity of the second stem cell without failing to collect the second stem cell from the second and the third culture containers with good timing, collects the second stem cell from the second and the third culture containers, and using the second stem cell, can assuredly manufacture a culture product liquid containing only a predetermined metabolite secreted from a single type of stem cell.

The method for manufacturing a culture product liquid, in which the second target ratio of the total area of the second stem cell is 88 to 92% of the bottom-surface area of the second culture container, and the third target ratio of the total area of the second stem cell is 88 to 92% of the bottom-surface area of the third culture container, gradually reduces the activity of the second stem cell when the total area of the second stem cell in relation to the bottom-surface area of the second and the third culture containers exceeds 92% to proliferate the second stem cell, and collects the second stem cell from the second and the third culture containers when the total area of the second stem cell in relation to the bottom-surface area of the second and the third culture containers increases to 88 to 92% for proliferation, thereby maintaining the activity of the second stem cell, accordingly proliferating the second stem cell, and assuredly manufacturing a culture product liquid containing only a predetermined metabolite secreted from a single type of second stem cell using the second stem cell.

In the method for manufacturing a culture product liquid, in which the first and the second stem cells are mesenchymal stem cells, using the single type of second stem cell cultured, various types of metabolites secreted from various types of stem cells are not contained, but only a predetermined metabolite secreted from the single type of second stem cell is contained, and a culture product liquid capable of providing the only specific effect on predetermined diseases, and predetermined cosmetic uses such as skin care, hair care, and body care can be manufactured, a culture product liquid capable of effective use in treating predetermined diseases can be manufactured, and a culture product liquid capable of effective use in predetermined cosmetic uses can be manufactured. The method for manufacturing a culture product liquid can manufacture a culture product liquid, using a pure (clear) second stem cell obtained by removing unnecessary stem cell, and allow the culture product liquid to contain only a predetermined metabolite secreted from a single type of second stem cell, thereby providing a significant effect of treating predetermined diseases and manufacturing a culture product liquid effective in curing such diseases, which makes it possible to manufacture a culture product liquid having a significant effect on predetermined cosmetic uses.

### BRIEF DESCRIPTION OF THE DRAWINGS

FIG. 1 is a schematic block diagram illustrating one example of a culture product liquid manufacturing system;
FIG. 2 is an explanatory drawing illustrating one example of a step of separating a bone marrow liquid;
FIG. 3 is an explanatory drawing illustrating a step of separating a bone marrow liquid following the step in FIG. 2;
FIG. 4 is an explanatory drawing illustrating a step of separating a bone marrow liquid following the step in FIG. 3;
FIG. 5 is an explanatory drawing illustrating one example of a step of first observing a stem cell;
FIG. 6 is a side view of a first flat culture container;
FIG. 7 is a partially enlarged view illustrating one example of a plane shape of a first mesenchymal stem cell;
FIG. 8 is a partially enlarged view illustrating another example of a plane shape of a first mesenchymal stem cell;
FIG. 9 is a partially enlarged view illustrating another example of a plane shape of a first mesenchymal stem cell;
FIG. 10 is an explanatory drawing illustrating one example of a step of separating a stem cell;
FIG. 11 is an explanatory drawing illustrating one example of a step of second extracting a stem cell;
FIG. 12 is an explanatory drawing illustrating one example of a step of third observing a stem cell;
FIG. 13 is a side view of a second flat culture container;
FIG. 14 is a partially enlarged view illustrating one example of a plane shape of a second mesenchymal stem cell;
FIG. 15 is a partially enlarged view illustrating another example of a plane shape of a second mesenchymal stem cell;
FIG. 16 is a partially enlarged view illustrating another example of a plane shape of a second mesenchymal stem cell;
FIG. 17 is a diagram illustrating one example of a culture product liquid extracted and a second mesenchymal stem cell stored;
FIG. 18 is an explanatory drawing illustrating one example of a step of fifth observing a stem cell; and
FIG. 19 is a side view illustrating a third flat culture container.

### DESCRIPTION OF THE PREFERRED EMBODIMENTS

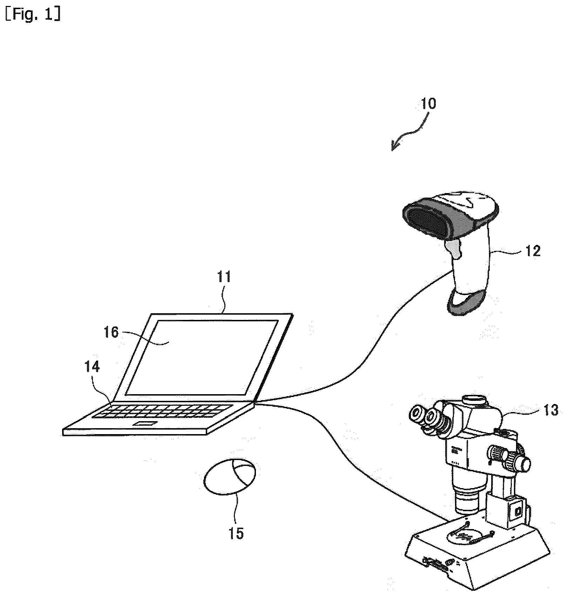

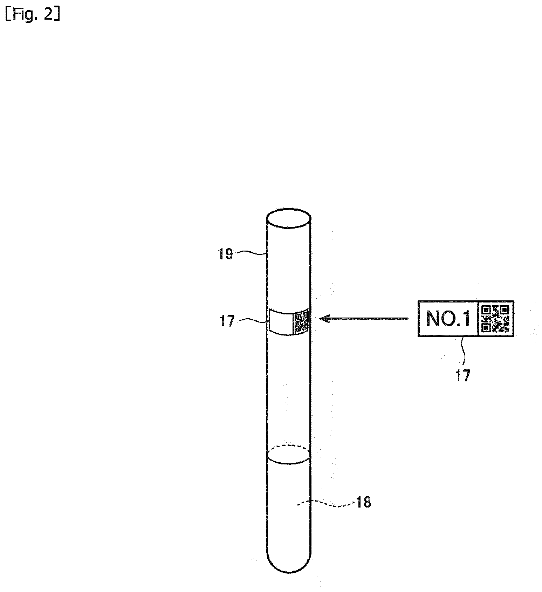

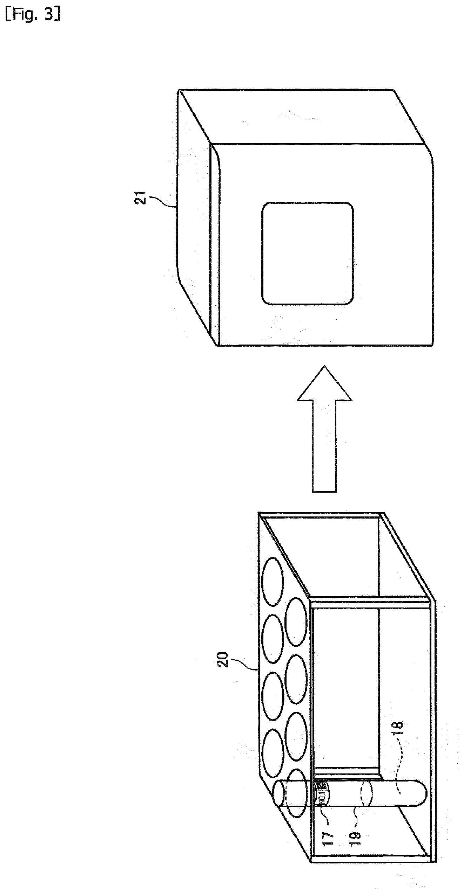

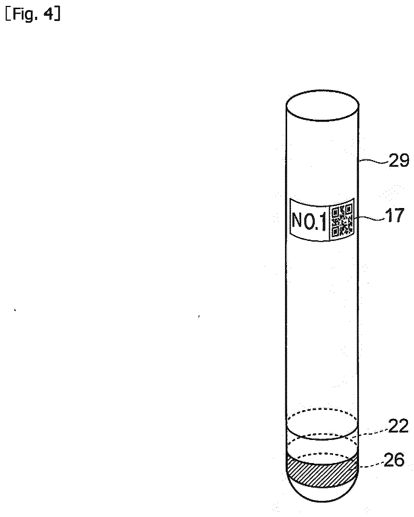

With reference to the accompanying drawings such as FIG. 1 illustrating a schematic block diagram of one example of a culture product liquid manufacturing system 10, the method for manufacturing a culture product liquid according to the present invention will be described as follows. FIG. 2 is an explanatory drawing illustrating one example of separating a bone marrow liquid, and FIG. 3 is an explanatory drawing illustrating a step of separating a bone marrow liquid separate following the step in FIG. 2. FIG. 4 is an explanatory drawing illustrating a step of separating a bone marrow liquid following the step in FIG. 3.

The method for manufacturing a culture product liquid cultures a single and specific type of mesenchymal stem cell (a single type of stem cell) among a plurality of types of mesenchymal stem cells contained in a bone marrow liquid, and manufactures a culture product liquid containing a predetermined metabolite secreted from the single type of mesenchymal stem cell. Specifically, the method for manufacturing a culture product liquid manufactures a culture product liquid, using a bone marrow liquid (raw bone marrow liquid) collected from a plurality of donors (humans) as a raw material and a culture product liquid manufacturing system 10, via a step of separating a bone marrow liquid separate, a step of extracting a bone marrow liquid, a step of first fixing a stem cell, a step of first culturing a stem cell, a step of first collecting a stem cell, a step of separating a stem cell, a step of second collecting a stem cell, a step of second fixing a stem cell, a step of second culturing a stem cell, a step of first extracting a culture product liquid. Further, using the culture product liquid manufacturing system 10, the culture product liquid is manufactured via a step of second collecting a stem cell, a step of third fixing a stem cell, a step of third culturing a stem cell, and a step of second extracting a culture product liquid.

The step of first fixing a stem cell includes a step of first observing a stem cell, and the step of second collecting a stem cell includes a step of second observing a stem cell. The step of second fixing a stem cell includes a step of third observing a stem cell, and the step of first extracting a culture product liquid includes a step of fourth observing a stem cell. The step of third fixing a stem cell includes a step of fifth observing a stem cell, and the step of second extracting a culture product liquid includes a step of sixth observing a stem cell.

The culture product liquid manufacturing system 10 is provided with a computer 11, a bar code reader 12, and an electron microscope 13. The computer 11, including a central processing unit (CPU or virtual CPU), a storage unit (memory or virtual memory), and a mass storage area (including hard disk or virtual hard disk), is operated by a physical OS (operating system) and a virtual OS (virtual operating system). With the computer 11 are connected input devices such as a keyboard 14 and a mouse 15 and output devices such as a display 16 and a printer (not shown) via an interface (wireless or wired).

The culture product liquid manufacturing system 10 employs QR code (registered trademark) to manage donor data (donor specific information). The donor data include donor's name, permanent address, telephone number, date of birth, gender, blood type, height, weight, and e-mail address. The system 10 employs QR code as a two-dimensional code, but additionally matrix SP code, Veri code, Maxi code, CP code, Data Matrix, Code1, Aztec code, intacta code, and Card e may be used. Other two-dimensional codes for use in the system 10 include all codes to be developed as such.

The step of separating a bone marrow liquid separate separates in layers a bone marrow liquid 18 collected from a donor. In the step of collecting a bone marrow liquid, 2 to 3cc (2 to 3ml) of the bone marrow liquid 18 is collected from the breastbone or ilium (pelvis) of the donor. The bone marrow liquid 18, after local anesthesia is used on the donor, is collected by "bone marrow puncture" (a.k.a. "MARK", originating in a German word, "Knochenmark", meaning bone marrow) for puncturing a bone marrow and sucking a bone marrow liquid (bone marrow blood). Those in charge such as doctors, nurses, and researchers, upon collection of the bone marrow liquid 18, activate the system 10 on the computer 11, and input donor data into the computer 11, using input devices such as the keyboard 14 and the mouse 15.

The computer 11 generates a unique donor identifier for identifying each donor each time donor data is input (the bone marrow liquid 18 is collected from the donor), and stores the donor data in a storage area so as to be associated with the donor identifier (step of storing donor data) . The computer 11 converts the input donor data into a QR code (two-dimensional code) by a two-dimensional code writing function (means of converting two-dimensional code (QR code)), outputs a first code paper 17 in which the QR code is printed (step of outputting two-dimensional code (QR code)), and stores the QR code in the storage area so as to be associated with the donor identifier for identifying each donor (step of storing two-dimensional code (QR code)). When second donor data is input, a second code paper in which a QR code is printed is output, and when a third donor data is input, a third code paper in which a QR code is printed is output, and accordingly first to nth code papers are output for different donors.

The QR code printed into the first code paper 17 in which NO1 is printed stores a NO. 1 indicative of a step of separating a bone marrow liquid, a step of extracting a bone marrow liquid, a step of first observing a stem cell, a step of first fixing a stem cell, a step of first culturing a stem cell, a step of second observing a stem cell, a step of first collecting a stem cell, a step of separating a stem cell, a step of second collecting a stem cell, a step of third observing a stem cell, a step of second fixing a stem cell, a step of second culturing a stem cell, a step of fourth observing a stem cell, a step of first extracting a culture product liquid, a step of second collecting a stem cell, a step of third fixing a stem cell, a step of third culturing a stem cell, a step of second extracting a culture product liquid, a step of second collecting a stem cell, a step of fifth observing a stem cell, a step of third fixing a stem cell, a step of third culturing a stem cell, and a step of sixth observing a stem cell, a step of second extracting a culture product liquid. The computer 11, each time the steps from the step of separating a bone marrow liquid to the step of first extracting a culture product liquid are taken, compares the donor data stored in the storage area with the donor data shown by the QR code read by the bar code reader 12.

2 to 3cc of a bone marrow liquid 18 collected from a donor, as illustrated in FIG. 2, is fed (stored) into a vertically elongating glass test tube 19 (separation container). 2 to 3cc of the bone marrow liquid 18 contains 0.5 to 1ml (approx. 5×10⁷ (cells/ml)) of a plurality of types of mesenchymal stem cells. On the outer peripheral surface of the glass test tube 19 for feeding the bone marrow liquid 18 is affixed the first code paper 17. The glass test tube 19 into which the bone marrow liquid 18 is fed, as illustrated in FIG. 3, is placed in a test tube rack 20 and is stored in a constant temperature bath 21 together with the test tube rack 20.

Those in charge such as doctors, nurses, and researchers, after affixing the first code paper 17 in which a QR code is printed to the outer peripheral surface of the glass test tube 19, allow the bar code reader 12 to read the QR code of the glass test tube 19. The bar code reader 12 is connected to the computer 11 via an interface (wired or wireless) . The bar code reader 12 transmits the read QR code to the computer 11.

The computer 11 extracts the NO. 1 shown by the QR code transmitted from the bar code reader 12 and the donor data from the storage area, reads it in a cache memory, and displays a step first display screen (not shown) on the display 16. The step first display screen displays the NO. 1 and the donor data, as well as a bone marrow liquid separation button, a bone marrow liquid extraction button, a stem cell first observation button, a stem cell second observation button, a stem cell first collection button, and a log-out button. The log-out button is clicked to stop the system 10 on the computer 11.

Those in charge, after clicking the bone marrow liquid separation button displayed on the display 16, feed the bone marrow liquid 18 from the injector into the glass test tube 19, and insert into the test tube rack 20 the glass test tube 19 into which the bone marrow liquid 18 is fed (placed). Those in charge, as illustrated in FIG. 3, store the test tube rack 20 in the constant temperature bath 21, and allow the glass test tube 19 into which the bone marrow liquid 18 is fed to statically stand in the constant temperature bath 21 for a predetermined period of time (approx. 2 hours) (statically without movement). The temperature in the constant temperature bath 21 is maintained at approximately the same as the body temperature of approx. 36 to 37°C. The computer 11 displays the NO. 1 shown by a QR code printed in the first code paper 17 and the donor data on the display 16, as well as a bone marrow liquid separation under-way message and a bone marrow liquid separation end button.

The glass test tube 19 is allowed to statically stand in the constant temperature bath 21 for a predetermined period of time (approx. 2 hours), as illustrated in FIG. 4, to vertically separate in layers the bone marrow liquid 18 fed into the test tube 20 in the test tube 20 (a step of separating a bone marrow liquid) . Culturing of a stem cell usually requires 150 to 200cc (150 to 200ml) of a bone marrow liquid, but the method for manufacturing a culture product liquid manufactures a single type of stem cell from 2 to 3cc of a bone marrow liquid 18 in small quantities collected from a donor, thereby only requiring collection of 2 to 3cc of a bone marrow liquid 18 in small quantities from the donor and minimizing the burden on the donor when the bone marrow liquid 18 is being collected.

The step of separating a bone marrow liquid for separating in layers the bone marrow liquid 18 is followed by the step of extracting a bone marrow liquid. In the step of extracting a bone marrow liquid, an intermediate-layer bone marrow liquid 22 is extracted from the bone marrow liquid 18 separated in layers. Those in charge, after confirming that the bone marrow liquid 18 is separated in layers, click the bone marrow liquid separation end button displayed on the display 16. When the bone marrow liquid separation end button is clicked, the computer 11 displays a step second display screen (not shown) on the display 16. On the step second display screen are displayed the NO. 1 and the donor data, as well as a bone marrow liquid separation end message, a bone marrow liquid extraction button, a stem cell first observation button, a stem cell second observation button, and a stem cell first collection button.

Those in charge click the bone marrow liquid extraction button on the step second display screen, and allow the bar code reader 12 to read the QR code of the glass test tube 19. When the QR code is transmitted from the bar code reader 12 to the computer 11, the computer 11 compares the donor data (donor data read in the memory) displayed on the display 16 with the donor data shown by the QR code read by the bar code reader 12, and these donor data are matched, the NO. 1 and the donor data are displayed on the display 16, as well as a bone marrow liquid separation end message, a bone marrow liquid extraction under-way message, and a bone marrow liquid extraction end button. When these donor data are not matched, the computer 11 displays an error message and a culture cancellation message on the display 16.

Those in charge take the test tube rack 20 out of the constant temperature bath 21, remove the glass test tube 19 from the test tube rack 20, and extract an intermediate-layer bone marrow liquid 22 present in the specific layer of the bone marrow liquid 18 separated in layers (a step of extracting a bone marrow liquid) . Those in charge extract (suck) an intermediate-layer bone marrow liquid 22, 2 to 4mm thick positioned in the intermediate layer of the bone marrow liquid 18 separated in layers, using an injector (not shown). Alternatively, those in charge extract (suck) an intermediate-layer bone marrow liquid 22, 2 to 4mm thick positioned in the intermediate layer of the bone marrow liquid 18 separated in layers, using a pipette (not shown).

The method for manufacturing a culture product liquid, after vertically separating in layers a bone marrow liquid 18 containing various types of mesenchymal stem cells, can assuredly extract a specific intermediate-layer bone marrow liquid 22 from the bone marrow liquid 18, using an injector or a pipette, and remove unnecessary mesenchymal stem cell contained in the bone marrow liquid 18.

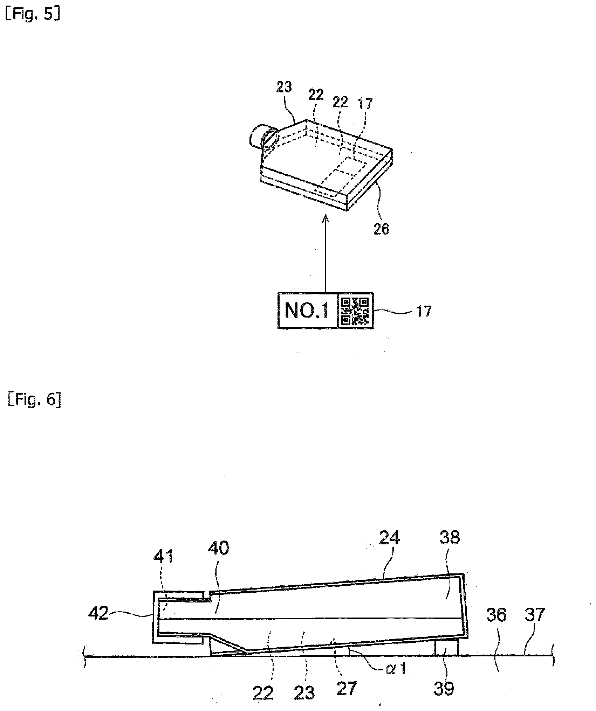

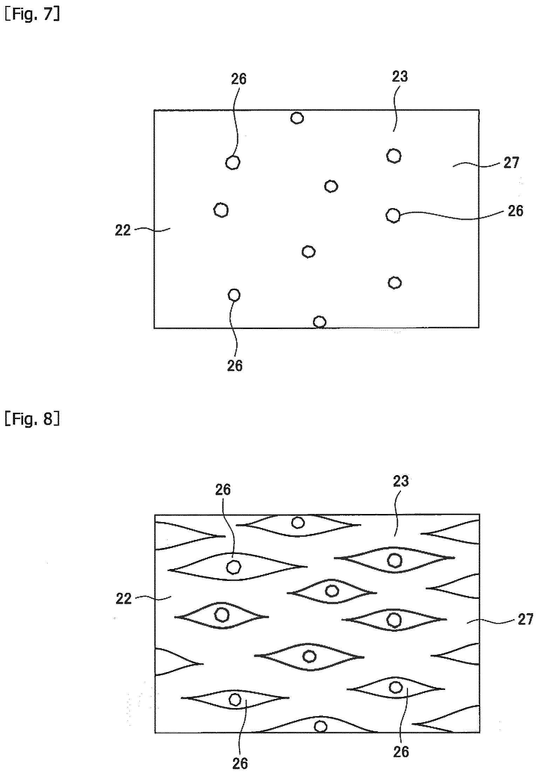

FIG. 5 is an explanatory drawing illustrating one example of a step of first observing a stem cell, and FIG. 6 is a side view illustrating a first flat culture container. FIG. 7 is a partially enlarged view illustrating one example of a plane shape of a first mesenchymal stem cell 26, and FIG. 8 is a partially enlarged view illustrating another example of a plane shape of a first mesenchymal stem cell 26. FIGS. 7 and 8 are magnified views of plane shapes of a first mesenchymal stem cell 26 taken with the electron microscope 13.

The step of extracting a bone marrow liquid for extracting from the bone marrow liquid 18 the specific intermediate-layer bone marrow liquid 22 positioned in the intermediate layer is followed by the step of first observing a stem cell. In the step of first observing a stem cell, the intermediate-layer bone marrow liquid 22 and the culture liquid 23 are fed (stored) into the first flat culture container 24 (first culture container), the culture container 24 is allowed to be maintained at approximately the same temperature as the body temperature (approx. 36 to 37°C) and to statically stand for 12 to 24 hours (statically without movement).

Those in charge such as doctors, nurses, and researchers, after extracting the specific intermediate-layer bone marrow liquid 22 from the bone marrow liquid 18, click the bone marrow liquid extraction end button displayed on the display 16. When the bone marrow liquid extraction end button is clicked, the computer 11 displays a step third display screen (not shown) on the display 16. The step third display screen displays the NO. 1 and the donor data, as well as a bone marrow liquid separation end message, a bone marrow liquid extraction end message, a stem cell first observation button, a stem cell second observation button, and a stem cell first collection button.

Those in charge affix the first code paper 17 in which a QR code is printed to the bottom 27 (outer surface of bottom wall) of the first flat culture container 24. Then, those in charge click the stem cell first observation button on the step third display screen, and allow the bar code reader 12 to read the QR code of the culture container 24. When the QR code is transmitted from the bar code reader 12 to the computer 11, the computer 11 compares the donor data (donor data read in the memory) displayed on the display 16 with the donor data shown by the QR code read by the bar code reader 12, and when these donor data are matched, the NO. 1 and the donor data are displayed on the display 16, as well as a bone marrow liquid separation end message, a bone marrow liquid extraction end message, a stem cell first observation under-way message, and a stem cell first observation end button. When these donor data are not matched, the computer 11 displays an error message and a culture cancellation message on the display 16.

Those in charge feed (store) into the culture container 24 the intermediate-layer bone marrow liquid 22 sucked into the injector or the pipette, and using the injector or the pipette, feed the culture liquid 23 into the culture container 24. The first flat culture container 24 (first culture container) is made of transparent glass or transparent plastics, and a flat container whose plane shape is approximately regular rectangle, having a small capacity and a bottom 27 of a predetermined area. The first flat culture container 24 includes a top portion 40, a bottom portion 38, and a feed hole 41 provided at the top portion 40. The feed hole 41 is sealed by a cap 42 in watertight manner. The first flat culture container 24 may be a flat container whose plane shape is circular or elliptical, having a small capacity and a bottom 27 of a predetermined area. The first flat culture container 24 used as a means of first observing a stem cell has a capacity of approx. 20 to 30cc (preferably 25cc), and a bottom-surface area of approx. 25 to 36mm². The culture container 24 has a side 5 to 6mm long.

The culture liquid 23 contains a mineral salt solution and an amino acid containing penicillin (approx. 100U/ml), amphotericin (approx. 100ng/ml), streptomycin (approx. 100mkg/ml), L-glutamine (approx. 2 to 4ml), and 20% fetal calf serum. The first mesenchymal stem cell 26 contained in the intermediate-layer bone marrow liquid 22 fed into the culture container 24 is fixed to the bottom 27 of the culture container 24 as time elapses, cultured by the culture liquid 23, and gradually proliferated (differentiated) on the bottom 27 of the culture container 24 to form a colony.

The culture liquid may be Dulbecco's Modified Eagle's Medium (DMEM), Grasgow Minimum Essential Medium (GMEM), RPMI640, and so on. The culture liquid may contain insulin, transferrin, ethanolamine, selenium, 2-mercaptoethanol, L-alanyl-L-glutamine, sodium pyruvate, L-alanine, L-asparagine, L-aspalathin acid, glycine, L-proline, and L-serine.

Those in charge, after feeding the intermediate-layer bone marrow liquid 22 and the culture liquid 23 into the first flat culture container 24, set (place) the culture container 24 in a specimen holder of the electron microscope 13. A spacer 39 is positioned between an upper surface 37 of a specimen holder 36 of the electron microscope 13 and a bottom portion 38 of the first flat culture container 24 so as to keep the bottom portion 38 of the culture container 24 raised by the spacer 39, allow the bottom portion 38 of the culture container 24 and the top portion 40 (feed hole 41) of the culture container 24 to stay at higher and lower positions, respectively, and maintain the culture container 24 at a predetermined tilted angle. Also, a spacer 39 may be positioned between the upper surface 37 of the specimen holder 36 of the electron microscope 13 and the top portion 40 of the first flat culture container 24 so as to keep the top portion 40 of the culture container 24 raised by the spacer 39, allow the top portion 40 of the culture container 24 and the bottom portion 38 of the culture container 24 to stay at higher and lower positions, respectively, and maintain the culture container 24 at a predetermined tilted angle. The tilted angle α1 of the first flat culture container 24 in relation to the upper surface 37 of the specimen holder 36 ranges from 2 to 5°, preferably 2 to 3°.

The method for manufacturing a culture product liquid places the first flat culture container 24 in relation to the upper surface 37 of the specimen holder 36 at the tilted angle, thereby allowing the intermediate bone marrow liquid 22 and the culture liquid 23 in the culture container 24 to tilt to the side of the top portion 40 of the culture container 24 (or the side of the bottom portion 38), causing the water pressure of the intermediate bone marrow liquid 22 and the culture liquid 23 to increase on the side of the top portion 49 of the culture container 24 (or side of the bottom portion 38), allowing the first mesenchymal stem cell 26 to concentrate on the side of the bottom portion 38 of the culture container 24, and thus increasing the activity of the first mesenchymal stem cells 26 to readily and immediately fix the first mesenchymal stem cell 26 on the bottom 27 of the culture container 24.

The electron microscope 13 is connected to the computer 11 via an interface (wired or wireless). The electron microscope 13 includes an imaging function for allowing an image sensor to image a magnified view of a subject and an image transmitting function for transmitting the magnified view to the computer 11. The electron microscope 13 images a magnified view of a plane shape of the first mesenchymal stem cell 26 contained in the intermediate-layer bone marrow liquid 22 fed into the culture container 24 at an interval of approx. 1 to 2 hours, and transmits the magnified view of the plane shape of the imaged stem cell 26 to the computer 11 at an interval of approx. 1 to 2 hours. The interval of imaging or image transmission in the electron microscope 13 can be set at 1 to 2 hours by input devices such as the keyboard 14 and the mouse 15.

The computer 11 stores a magnified view of a plane shape of the first mesenchymal stem cell 26 transmitted from the electron microscope 13 and an imaging time in the storage area so as to be associated with the donor identifier (step of first storing a stem cell image) . The computer 11 displays the magnified view of the plane shape of the stem cell 28 transmitted from the electron microscope 13 and the imaging time on the display 16. Those in charge confirm (visually recognize) the magnified view of the plane shape of the stem cell 26 displayed on the display 16 at an interval of approx. 1 to 2 hours for 12 to 24 hours, and observe changes in the plane shape of the stem cell 26 contained in the intermediate-layer bone marrow liquid 22 (step of first observing a stem cell) . Those in charge may directly observe changes in the plane shape of the first mesenchymal stem cell 26 from the observation window of the electron microscope 13 at an interval of approx. 1 to 2 hours for 12 to 24 hours.

The method for manufacturing a culture product liquid cultures the first mesenchymal stem cell 26 contained in the intermediate-layer bone marrow liquid and fixes the first mesenchymal stem cell 26 to the bottom 27 of the first flat culture container 24 (first culture container) (step of first fixing a stem cell) . The initial plane shape of the first mesenchymal stem cell 26 is an approximately circular shape, and when the plane shape of the stem cell 26 is the approximately circular shape, the stem cell 26 is not fixed to the bottom 27 of the culture container 24 (inner surface of bottom wall), which doesn't allow the stem cell 26 to start proliferation (differentiation). The plane shape of the first mesenchymal stem cell 26 deformed is primarily the approximately circular shape before fixing, and a flat shape when the stem cell 26 elongates (expands) in amorphous shape in one direction (predetermined direction), thereby fixing the stem cell 26 to the bottom 27 of the culture container 24 (inner surface of bottom wall) and allowing the stem cell 26 to start proliferation (differentiation).

Those in charge, according to the observation in the step of first observing a stem cell, as illustrated in FIG. 6, when the magnified view of the plane shape of the first mesenchymal stem cell 26 displayed on the display 16 remains observed as an approximately circular shape, determine that the stem cell 26 is not fixed to the bottom 27 of the culture container 24 (inner surface of bottom wall), and continuously observe changes in the plane shape of the stem cell 26 at an interval of approx. 1 to 2 hours. Those in charge, according to the observation in the step of first observing a stem cell, as illustrated in FIG. 7, when the plane shape of the first mesenchymal stem cell 26 displayed on the display 16 is deformed from the approximately circular to the primarily approximately circular and amorphous flat shape, determine that the stem cell 26 is fixed to the bottom 27 of the culture container 24.

The use of a large culture container in which the capacity exceeds 30cc to fix the first mesenchymal stem cell 26 and the bottom-surface area exceeds 36mm² fails to readily fix the stem cell 26 to the bottom surface of the container and slows down the proliferation of the stem cell 26, but the method for manufacturing a culture product liquid, using the first flat culture container 24 having the capacity and the bottom-surface area, can readily fix the stem cell 26 to the bottom 27 of the culture container 24 and immediately proliferate the stem cell 26 in the culture container 24.

The method for manufacturing a culture product liquid allows the culture container 24 to statically stand at approximately the same temperature as the body temperature for a period of 12 to 24 hours, and observes the deformation of the first mesenchymal stem cell 26 from the initial plane shape in the culture container 24 at an interval of approx. 1 to 2 hours for the period of 12 to 24 hours, thereby never failing to confirm deformation of the stem cell 26 and properly confirming the fixing of the stem cell 26 to the bottom 27 of the culture container 24.

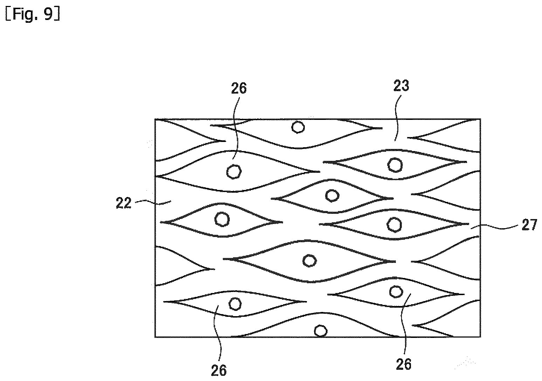

FIG. 9 is a partially enlarged view illustrating another example of a plane shape of a first mesenchymal stem cell 26. FIG. 9 is a magnified view of a plane shape of a first mesenchymal stem cell 26 imaged by an electron microscope 13. According to the observation in the step of first observing a stem cell step, the first mesenchymal stem cell 26 (first stem cell) is deformed from the approximately circular (initial plane shape) to the primarily approximately circular and amorphous flat shape, and the step of first fixing a stem cell for confirming the fixing of the stem cell 26 to the bottom 27 of the first flat culture container 24 (first culture container) is followed by the step of first culturing a stem cell and the step of second observing a stem cell.

In the step of first culturing a stem cell and the step of second observing a stem cell, the culture liquid 23 fed into the culture container 24 is discharged (discarded) from the culture container 24, and a new culture liquid 23 is fed (stored) into the culture container 24. Then, the culture container 24 is allowed to statically stand at approximately the same temperature as the body temperature (approx. 36 to 37°C) for 36 to 48 hours (statically without movement), and cultures the stem cell 26 until the total area of the first mesenchymal stem cell 26 reaches a first target ratio of the bottom-surface area of the culture container 24 (step of first culturing a stem cell) .

The total area of the first mesenchymal stem cell 26 fixed to the bottom 27 of the culture container 24, by allowing the culture container 24 to statically stand at an interval of approx. 1 to 2 hours for 36 to 48 hours, in relation to the bottom-surface area of the culture container 24, is observed with the electron microscope 13, and it is determined whether the total area of the stem cell 26 reaches the first target ratio of the bottom-surface area of the culture container 24. The first target ratio of the total area of the first mesenchymal stem cell 26 is 70 to 80% of the bottom-surface area of the culture container 24 (70 to 80% confluent).

Those in charge such as doctors, nurses, and researchers, after confirming the fixing of the first mesenchymal stem cell 26 contained in the intermediate-layer bone marrow liquid 22 to the bottom 27 of the culture container 24, click the stem cell first observation end button displayed on the display 16. When the stem cell first observation end button is clicked, the computer 11 displays a step fourth display screen (not shown) on the display 16. The step fourth display screen displays the NO. 1 and the donor data, as well as a bone marrow liquid separation end message, a bone marrow liquid extraction end message, a stem cell first observation end message, a stem cell second observation button, and a stem cell first collection button.

Those in charge click the stem cell second observation button on the step fourth display screen, remove the culture container 24 from the specimen holder of the electron microscope 16, and allow the bar code reader 12 to read the QR code of the culture container 24. When the QR code is transmitted from the bar code reader 12 to the computer 11, the computer 11 compares the donor data (donor data read in memory) displayed on the display 16 with the donor data shown by the QR code read by the bar code reader 12, and when these donor data are matched, the NO. 1 and the donor data are displayed on the display 16, as well as a bone marrow liquid separation end message, a bone marrow liquid extraction end message, a stem cell first observation end message, a stem cell second observation under-way message, and a stem cell second observation end button. When these donor data are not matched, the computer 11 displays an error message and a culture cancellation message on the display 16.

Those in charge discharge (discard) the culture liquid 23 fed into the culture container 24 in the step of first observing a stem cell step from the culture container 24, and feed (store) a new culture liquid 23 into the culture container 24. The new culture liquid 23 is the same as that fed in the step of first observing a stem cell. Those in charge feed a new culture liquid 23 into the culture container 24, and then set (place) the culture container 24 in the specimen holder of the electron microscope 13.

A spacer 39 is positioned between an upper surface 37 of a specimen holder 36 of the electron microscope 13 and a bottom portion 38 of the first flat culture container 24 so as to keep the bottom portion 38 of the culture container 24 raised by the spacer 39, allow the bottom portion 38 of the culture container 24 and the top portion 40 (feed hole 41) of the culture container 24 to stay at higher and lower positions, respectively, and maintain the culture container 24 at a predetermined tilted angle (see FIG. 6). Also, a spacer 39 may be positioned between the upper surface 37 of the specimen holder 36 of the electron microscope 13 and the top portion 40 of the first flat culture container 24 so as to keep the top portion 40 of the culture container 24 raised by the spacer 39, allow the top portion 40 of the culture container 24 and the bottom portion 38 of the culture container 24 to stay at higher and lower positions, respectively, and maintain the culture container 24 at a predetermined tilted angle. The tilted angle α1 of the first flat culture container 24 in relation to the upper surface 37 of the specimen holder 36 ranges from 2 to 5°, preferably 2 to 3°.

The method for manufacturing a culture product liquid confirms the fixing of the first mesenchymal stem cell 26, then discharges the culture liquid 23 in the culture container 24, and feeds a new culture liquid 23 into the culture container 24, thereby assuredly encouraging the proliferation of the stem cell 26. The method for manufacturing a culture product liquid places the first flat culture container 24 in relation to an upper surface 37 of the specimen holder 36 at the tilted angle, thereby allowing the first mesenchymal stem cell 26 and the culture liquid 23 in the culture container 24 to tilt to the side of the top portion 40 of the culture container 24 (or the side of the bottom portion 38), causing the water pressure of the first mesenchymal stem cell 26 and the culture liquid 23 to increase on the side of the top portion 40 of the culture container 24 (or side of the bottom portion 38), allowing the first mesenchymal stem cell 26 to concentrate on the side of the bottom portion 38 of the culture container 24, and thus increasing the activity of the first mesenchymal stem cells 26 to readily and immediately proliferate (differentiate) the first mesenchymal stem cell 26 on the bottom 27 of the culture container 24.

The electron microscope 13 images a magnified view of a plane shape of the first mesenchymal stem cell 26 in the culture container 24 at an interval of approx. 1 to 2 hours, and transmits the magnified view of the plane shape of the imaged stem cell 26 to the computer 11 at an interval of approx. 1 to 2 hours. The computer 11 stores the magnified view of the plane shape of the stem cell 26 transmitted from the electron microscope 13 and an imaging time in the storage area so as to be associated with the donor identifier (step of second storing a stem cell image) . The computer 11 displays the magnified view of the plane shape of the stem cell 26 transmitted from the electron microscope 13 and the imaging time on the display 16.

Those in charge confirm (visually recognize) the magnified view of the plane shape of the first mesenchymal stem cell 26 displayed on the display 16 at an interval of approx. 1 to 2 hours for 36 to 48 hours, observe the total area of the stem cell 26 fixed to the bottom 27 of the culture container 24 in relation to the bottom-surface area of the culture container 24, and determine whether the total area of the stem cell 26 reaches the first target ratio of the bottom-surface area of the culture container 24 (70 to 80% confluent) (step of second observing a stem cell) . Those in charge may directly observe the total area of the first mesenchymal stem cell 26 in relation to the bottom-surface area of the culture container 24 from the observation window of the electron microscope 13 at an interval of approx. 1 to 2 hours for 36 to 48 hours, and determine whether the total area of the stem cell 26 reaches the first target ratio of the bottom-surface area of the culture container 24 (70 to 80% confluent).

Those in charge, according to the observation in the step of second observing a stem cell, as illustrated in FIG. 7, when the total area of the first mesenchymal stem cell 26 displayed on the display 16 doesn't reach the first target ratio of the bottom-surface area of the culture container 24 (70 to 80% confluent), continuously observe the total area of the stem cell 26 in relation to the bottom-surface area of the culture container 24 at an interval of approx. 1 to 2 hours. When the total area of the first mesenchymal stem cell 26 reaches the first target ratio of all the area of the magnified view displayed on the display 16, it is determined that the total area of the stem cell 26 reaches the first target ratio of the bottom-surface area of the culture container 24.

According to the observation in the step of second observing a stem cell, the first mesenchymal stem cell 26 proliferates on the bottom 27 (inner surface of bottom wall) of the culture container 24 to form a colony and expands its plane shape, and as illustrated in FIG. 8, when the total area of the stem cell 26 displayed on the display 16 reaches the first target ratio of the bottom-surface area of the culture container 24 (70 to 80% confluent), a step of first collecting a stem cell for collecting the stem cell 26 from the culture container 24 is taken. The step of first collecting a stem cell, upon the total area of the first mesenchymal stem cell 26 reaching the first target ratio, collects the first mesenchymal stem cell 26 proliferated (differentiated) in the culture container 24 from the culture container 24.

Those in charge such as doctors, nurses, and researchers, after confirming that the total area of the first mesenchymal stem cell 26 reaches the first target ratio of the bottom-surface area of the culture container 24, click the stem cell second observation end button displayed on the display 16. When the stem cell second observation end button is clicked, the computer 11 displays a step fifth display screen (not shown) on the display 16. The step fifth display screen displays the NO. 1 and the donor data, as well as a bone marrow liquid separation end message, a bone marrow liquid extraction end message, a stem cell first observation end message, a stem cell second observation end message, and a stem cell first collection button.

Those in charge click the stem cell first collection button on the step fifth display screen, and allow the bar code reader 12 to read the QR code of the culture container 24. When the QR code is transmitted from the bar code reader 12 to the computer 11, the computer 11 compares the donor data (donor data read in memory) displayed on the display 16 with the donor data shown by the QR code read by the bar code reader 12, and when these donor data are matched, the NO. 1 and the donor data are displayed on the display 16, as well as a bone marrow liquid separation end message, a bone marrow liquid extraction end message, a stem cell first observation end message, a stem cell second observation end message, a stem cell first collection under-way message, and a stem cell first collection end button. When these donor data are not matched, the computer 11 displays an error message and a culture cancellation message on the display 16.

Those in charge discharge (discard) the culture liquid 23 fed into the culture container 24 from the culture container 24 in the step of second observing a stem cell, using an injector or a pipette, and after washing the culture container 24 with PBS, feed a trypsin solution sucked in the injector or the pipette into the culture container 24. When the trypsin solution is fed into the culture container 24, the first mesenchymal stem cell 26 fixed to the bottom 27 of the culture container 24 peels off the bottom 27 by the trypsin solution, floating up to the surface of the trypsin solution. Those in charge collect (suck) the stem cell 26 using a pipette, and store the stem cell 26 in the pipette (step of first collecting a stem cell).

The method for manufacturing a culture product liquid observes the total area at an interval of approx. 1 to 2 hours to properly confirm the total area of the first mesenchymal stem cell 26 in relation to the bottom-surface area of the culture container 24, thereby assuredly confirming that the total area of the stem cell 26 reaches the first target ratio of the bottom-surface area of the culture container 24.

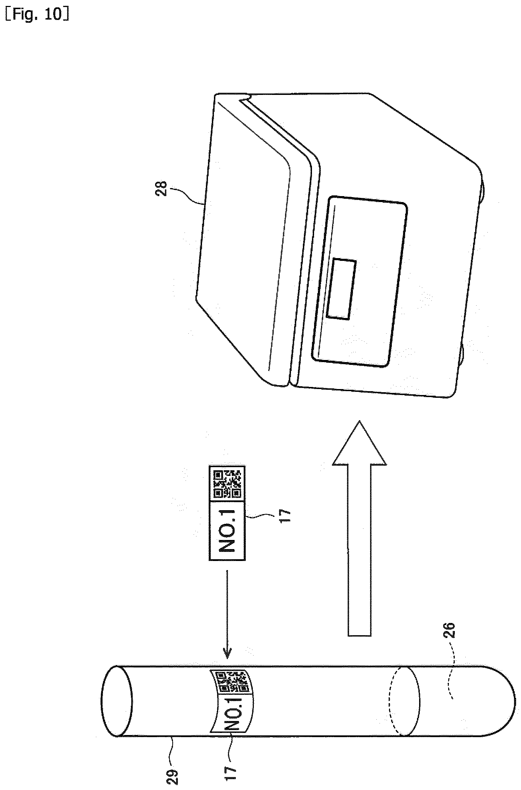

FIG. 10 is an explanatory drawing illustrating one example of a step of separating a stem cell. The step of first collecting a stem cell for collecting the first mesenchymal stem cell 26 from the culture container 24 is followed by a step of separating a stem cell. The step of separating a stem cell centrifugally separates in layers the first mesenchymal stem cell 26 collected by a means of first collection by the centrifugal separator 28. Those in charge such as doctors, nurses, and researchers, after sucking the first mesenchymal stem cell 26 from the culture container 24 in a pipette, click the stem cell first collection end button displayed on the display 16.

When the stem cell first collection end button is clicked, the computer 11 displays a step sixth display screen (not shown) on the display 16. The step sixth display screen displays the NO. 1 and the donor data, as well as a stem cell separation button, a stem cell second extraction button, a stem cell third observation button, a stem cell fourth observation button, and a first extraction button. Those in charge affix the first code paper 17 in which a QR code is printed to the outer peripheral surface of the glass test tube 29 for feeding the first mesenchymal stem cell 26.

Then, those in charge click the stem cell separation button on the step sixth display screen, and allow the bar code reader 12 to read the QR code of the glass test tube 29. When the QR code is transmitted from the bar code reader 12 to the computer 11, the computer 11 compares the donor data (donor data read in memory) displayed on the display 16 with the donor data shown by the QR code read by the bar code reader 12, and when these donor data are matched, the NO. 1 and the donor data are displayed on display 16, as well as a stem cell separation under-way message and a stem cell separation end button. When these donor data are not matched, the computer 11 displays an error message and a culture cancellation message on the display 16.

Those in charge feed (store) the first mesenchymal stem cell 26 in a pipette into the glass test tube 29, and place (set) the glass test tube 29 in the centrifugal separator 28. Those in charge, after centrifugally separating the stem cell 26 by the centrifugal separator 28 for a predetermined period of time, take the glass test tube 29 out of the centrifugal separator 28. The first mesenchymal stem cell 26 in the glass test tube 29 is vertically separated in layers by the centrifugal separator 28 (step of separating a stem cell).

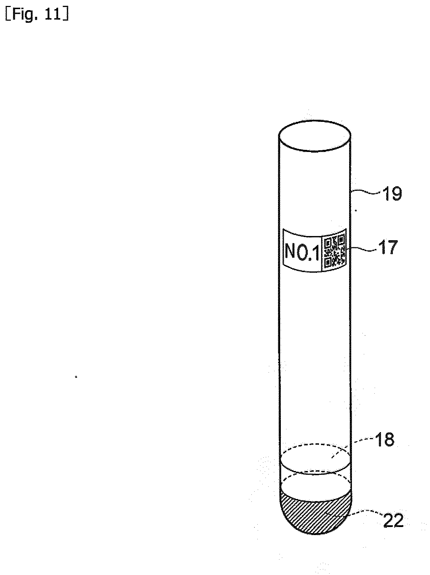

FIG. 11 is an explanatory drawing illustrating one example of a step of second collecting a stem cell. The step of separating a stem cell for separating in layers the first mesenchymal stem cell 26 is followed by a step of second collecting a stem cell. The step of second collecting a stem cell collects the second mesenchymal stem cell 30 positioned in the lower layer (lowermost layer) from the first mesenchymal stem cell 26 separated in layers. Those in charge such as doctors, nurses, and researchers, after vertically separating in layers the first mesenchymal stem cell 26 by the centrifugal separator 28, remove the glass test tube 29 from the centrifugal separator 28, and click the stem cell separation end button displayed on the display 16.

When the stem cell separation end button is clicked, the computer 11 displays a step seventh display screen (not shown) on the display 16. The step seventh display screen displays the NO. 1 and the donor data, as well as a stem cell separation end message, a stem cell second collection button, a stem cell third observation button, a stem cell fourth observation button, and a culture product liquid first extraction button.

Those in charge click the stem cell second collection button on the step seventh display screen, and allow the bar code reader 12 to read the QR code of the glass test tube 29. When the QR code is transmitted from the bar code reader 12 to the computer 11, the computer 11 compares the donor data (donor data read in memory) displayed on the display 16 with the donor data shown by the QR code read by the bar code reader 12, and when these donor data are matched, the NO. 1 and the donor data are displayed on the display 16, as well as a stem cell separation end message, a stem cell second collection under-way message, and a stem cell second collection end button. When these donor data are not matched, the computer 11 displays an error message and a culture cancellation message on the display 16.

Those in charge collect the second mesenchymal stem cell 30 present in the lower layer (lowermost layer) of the first mesenchymal stem cell 26 separated in layers in the glass test tube 29 (step of second collecting a stem cell). Those in charge collect (suck) the second mesenchymal stem cell 30 positioned in the lower layer (lowermost layer) of the first mesenchymal stem cell 26 separated in layers using an injector. Alternatively, those in charge collect (suck) the second mesenchymal stem cell 30 positioned in the lower layer (lowermost layer) of the first mesenchymal stem cell 26 separated in layers using the pipette.

The method for manufacturing a culture product liquid centrifugally separates the first mesenchymal stem cell 26 containing unnecessary stem cell with the centrifugal separator 28 to be vertically separated in layers and collects the second mesenchymal stem cell 30 positioned in the lower layer (lowermost layer) of the stem cell 26 centrifugally separated in layers, thereby assuredly collecting a specific second mesenchymal stem cell 30 from the stem cell 26 and removing unnecessary mesenchymal stem cell from the stem cell 26.

The method for manufacturing a culture product liquid gradually reduces the activity of the stem cell 26 when the total area of the first mesenchymal stem cell 26 in relation to the bottom-surface area of the first flat culture container 24 (first culture container) exceeds 80% to proliferate the stem cell 26, and collects the stem cell 26 from the culture container 24 when the total area of the stem cell 26 in relation to the bottom-surface area of the culture container 24 increases to 70 to 80% for proliferation, thereby maintaining the activity of the stem cell 26, accordingly proliferating the stem cell 26, and collecting the second mesenchymal stem cell 30 having activity from the stem cell 26.

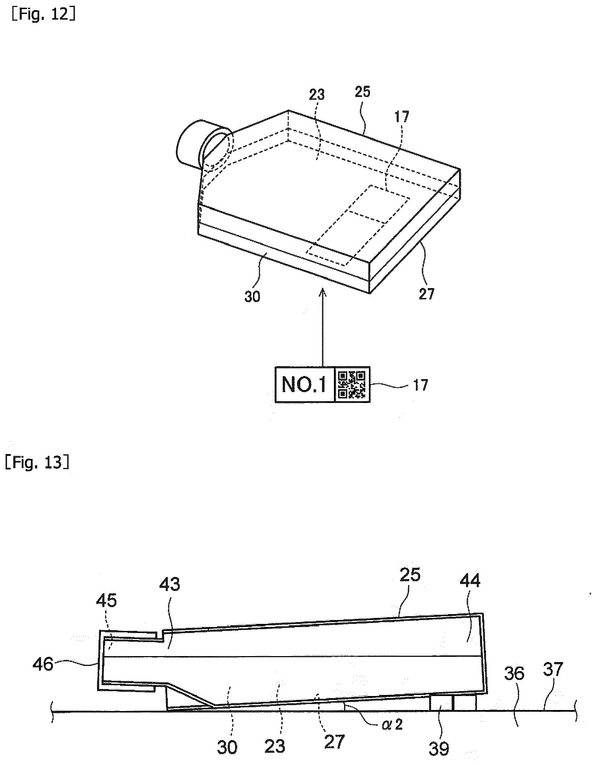

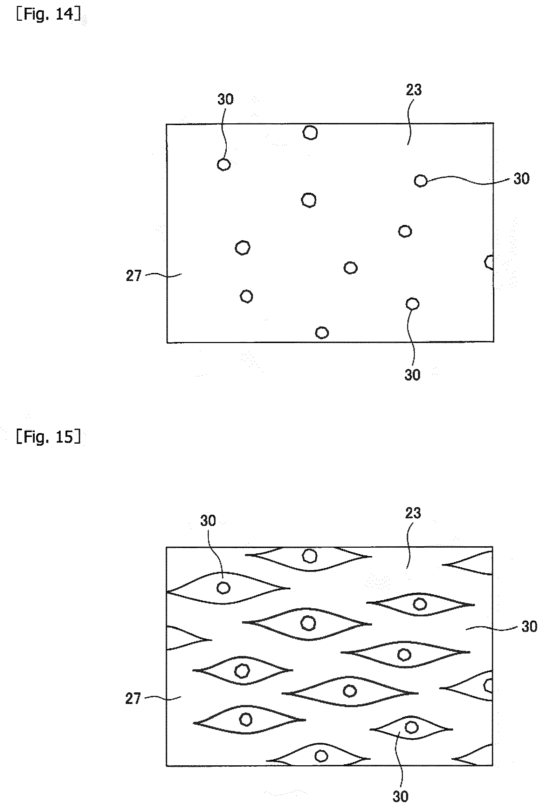

FIG. 12 is an explanatory drawing illustrating one example of a step of third observing a stem cell contained in a means of second fixing, and FIG. 13 is a side view showing a second flat culture container. FIG. 14 is a partially enlarged view illustrating one example of a plane shape of a second mesenchymal stem cell 30, and FIG. 15 is a partially enlarged view illustrating another example of a plane shape of a second mesenchymal stem cell 30. FIGS. 14 and 15 are magnified views illustrating plane shapes of a second mesenchymal stem cell 30 imaged by an electron microscope 13.

The step of second collecting a stem cell for collecting a specific second mesenchymal stem cell 30 positioned in the lower layer (lowermost layer) from the first mesenchymal stem cell 26 is followed by a step of third observing a stem cell and a step of second fixing a stem cell. In the step of third observing a stem cell and the step of second fixing a stem cell, the second mesenchymal stem cell 30 and the culture liquid 23 are fed (stored) into the second flat culture container 25 (second culture container), and the culture container 25 is allowed to statically stand (statically without movement) at approximately the same temperature as the body temperature (approx. 36 to 37°C) for 36 to 48 hours.

The deformation of the second mesenchymal stem cell 30 (second stem cell) from the initial plane shape in the culture container 25, by allowing the culture container 25 to statically stand at an interval of approx. 1 to 2 hours for 36 to 48 hours, is observed with the electron microscope 13, and it is determined whether the stem cell 30 is fixed to the bottom 27 of the culture container 25. To the bottom 27 (outer surface of bottom wall) of the culture container 25 into which the second mesenchymal stem cell 30 and the culture liquid 23 are fed is affixed the first code paper 17 in which a QR code for identifying a donor is printed.

Those in charge such as doctors, nurses, and researchers, after collecting a specific second mesenchymal stem cell 30 from the first mesenchymal stem cell 26, click the stem cell second collection extraction end button displayed on the display 16. When the stem cell second collection end button is clicked, the computer 11 displays a step eighth display screen (not shown) on the display 16. The step eighth display screen displays the NO. 1 and the donor data, as well as a stem cell separation end message, a stem cell second collection end message, a stem cell third observation button, a stem cell fourth observation button, and a culture product liquid first extraction button.

Those in charge affix the first code paper 17 in which a QR code is printed to the bottom 27 of the second flat culture container 25 (outer surface of bottom wall) . Then, those in charge click the stem cell third observation button on the step eighth display screen, and allow the bar code reader 12 to read the QR code of the culture container 25. When the QR code is transmitted from the bar code reader 12 to the computer 11, the computer 11 compares the donor data displayed on the display 16 (donor data read in memory) with the donor data shown by the QR code read by the bar code reader 12, and when these donor data are matched, the NO. 1 and the donor data are displayed on the display 16, as well as a stem cell separation end message, a stem cell second collection end message, a stem cell third observation under-way message, and a stem cell third observation end button. When these donor data are not matched, the computer 11 displays an error message and a culture cancellation message on the display 16.

Those in charge feed (store) the second mesenchymal stem cell 30 sucked in the injector or the pipette into the culture container 25, and feed (store) the culture liquid 23 in the culture container 25. The second flat culture container 25 (second culture container) is made of transparent glass or transparent plastics, and a flat container whose plane shape is approximately rectangle, having a predetermined capacity and a bottom 27 of a predetermined area, and the area of the bottom 27 is approx. twice as large as the first flat culture container 24 (first culture container). The second flat culture container 25 includes a top portion 43, a bottom portion 44, and a feed hole 45 provided at the top portion 43. The feed hole 45 is sealed by a cap 46 in watertight manner. The second flat culture container 25 may be a flat container whose plane shape is circular or elliptical, having a predetermined capacity and a bottom 27 of a predetermined area.

The second flat culture container 25 (second culture container) used in the step of third observing a stem cell has a capacity of approx. 40 to 60cc (preferably 50cc), and a bottom-surface area of approx. 50 to 72mm². The culture container 25 has a side of approx. 7 to 8.5mm long. The culture liquid 23 is the same as that fed in the step of first observing a stem cell. The second mesenchymal stem cell 30 fed into the culture container 25 is fixed to the bottom 27 of the culture container 25 as time elapses, cultured by the culture liquid 23, and gradually proliferated (differentiated) on the bottom 27 of the culture container 25 to form a colony.

Those in charge, after feeding the second mesenchymal stem cell 30 and the culture liquid 23 into the second flat culture container 25, set (place) the culture container 25 in a specimen holder 36 of the electron microscope 13. A spacer 39 is positioned between an upper surface 37 of a specimen holder 36 of the electron microscope 13 and a bottom portion 44 of the second flat culture container 25 so as to keep the bottom portion 44 of the culture container 25 raised by the spacer 39, allow the bottom portion 44 of the culture container 25 and the top portion 43 of the culture container 25 (feed hole 45) to stay at higher and lower positions, respectively, and maintain the culture container 25 at a predetermined tilted angle. Also, a spacer 39 may be positioned between the upper surface 37 of the specimen holder 36 of the electron microscope 13 and the top portion 43 of the second flat culture container 25 so as to keep the top portion 43 of the culture container 25 raised by the spacer 39, allow the top portion 43 of the culture container 25 and the bottom portion 44 of the culture container 25 to stay at higher and lower positions, respectively, and maintain the culture container 25 maintained at a predetermined tilted angle. The tilted angle α2 of the second flat culture container 25 in relation to the upper surface 37 of the specimen holder 36 ranges from 2 to 5°, preferably 2 to 3°.

The method for manufacturing a culture product liquid places the second flat culture container 25 in relation to the upper surface 37 of the specimen holder 36 at the tilted angle, thereby allowing the second mesenchymal stem cell 30 and the culture liquid 23 in the culture container 25 to tilt to the side of the top portion 43 of the culture container 25 (or the side of the bottom portion 44), causing the water pressure of the second mesenchymal stem cell 30 and the culture liquid 23 to increase on the side of the top portion 43 of the culture container 25 (or the side of the bottom portion 44), allowing the second mesenchymal stem cell 30 to concentrate on the side of the bottom portion 44 of the culture container 25, and thus increasing the activity of the second mesenchymal stem cells 30 to readily and immediately fix the second mesenchymal stem cell 30 to the bottom 27 of the culture container 25.

The electron microscope 13 images a magnified view of a plane shape of the second mesenchymal stem cell 30 fed into the culture container 25 at an interval of approx. 1 to 2 hours, and transmits the magnified view of the plane shape of the imaged stem cell 30 to the computer 11 at an interval of approx. 1 to 2 hours. The computer 11 stores a magnified view of a plane shape of the second mesenchymal stem cell 30 transmitted from the electron microscope 13 and an imaging time so as to be associated with the donor identifier (step of third storing a stem cell image) . The computer 11 displays the magnified view of the plane shape of the stem cell 30 transmitted from the electron microscope 13 and the imaging time on the display 16. Those in charge confirm (visually recognize) the magnified view of the plane shape of the stem cell 30 displayed on the display 16 at an interval of approx. 1 to 2 hours for 36 to 48 hours, and observe changes in the plane shape of the stem cell 30 (step of third observing a stem cell) . Those in charge may directly observe changes in the plane shape of the stem cell 30 from the observation window of the electron microscope 13 at an interval of approx. 1 to 2 hours for 36 to 48 hours.

The initial plane shape of the second mesenchymal stem cell 30 is an approximately circular shape, and when the plane shape of the stem cell 30 is the approximately circular shape, the stem cell 30 is not fixed to the bottom 27 of the culture container 25 (inner surface of bottom wall), which doesn't allow the stem cell 30 to start proliferation (differentiation). The plane shape of the second mesenchymal stem cell 30 deformed is primarily the approximately circular shape before fixing, and a flat shape when the stem cell 30 elongates in amorphous form in one direction, thereby fixing the stem cell 30 to the bottom 27 of the culture container 25 (inner surface of bottom wall) and allowing the stem cell 30 to start proliferation (differentiation).

Those in charge, according to the observation in the step of third observing a stem cell, as illustrated in FIG. 12, when the plane shape of the second mesenchymal stem cell 30 displayed on the display 16 remains observed as an approximately circular shape, determine that the stem cell 30 is not fixed to the bottom 27 of the culture container 25 (inner surface of bottom wall), and continuously observe changes in the plane shape of the stem cell 30 at an interval of approx. 1 to 2 hours. Those in charge, according to the observation in a means of third observing a stem cell, as illustrated in FIG. 13, when the plane shape of the second mesenchymal stem cell 30 displayed on the display 16 is deformed from the approximately circular to the primarily approximately circular and amorphous flat shape, determine that the stem cell 30 is fixed to the bottom 27 of the culture container 25 (step of second fixing a stem cell).

The use of a large culture container in which the capacity exceeds 60cc to fix the second mesenchymal stem cell 30 and the bottom-surface area exceeds 72mm² fails to readily fix the stem cell 30 to the bottom surface of the container and slows down the proliferation of the stem cell 30, but the method for manufacturing a culture product liquid, using the second flat culture container 25 having the capacity and the bottom-surface area, can readily fix the stem cell 30 to the bottom 27 of the culture container 25 and immediately proliferate the stem cell 30 in the culture container 25.

The method for manufacturing a culture product liquid allows the culture container 25 to statically stand at approximately the same temperature as the body temperature for 36 to 48 hours, and observes the deformation of the second mesenchymal stem cell 30 from the initial plane shape in the culture container 25 at an interval of approx. 1 to 2 hours for 36 to 48 hours, thereby never failing to confirm the deformation of the stem cell 30 and properly confirming the fixing of the stem cell 30 to the bottom 27 of the culture container 25.

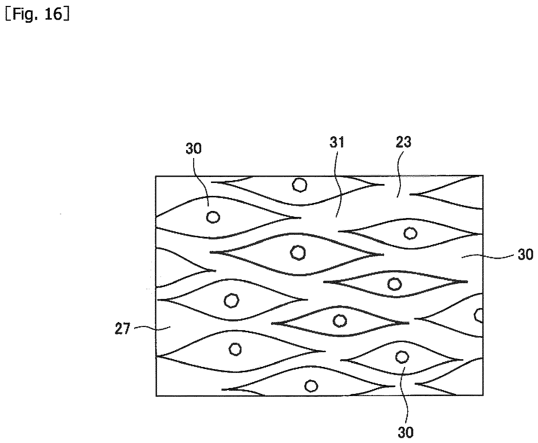

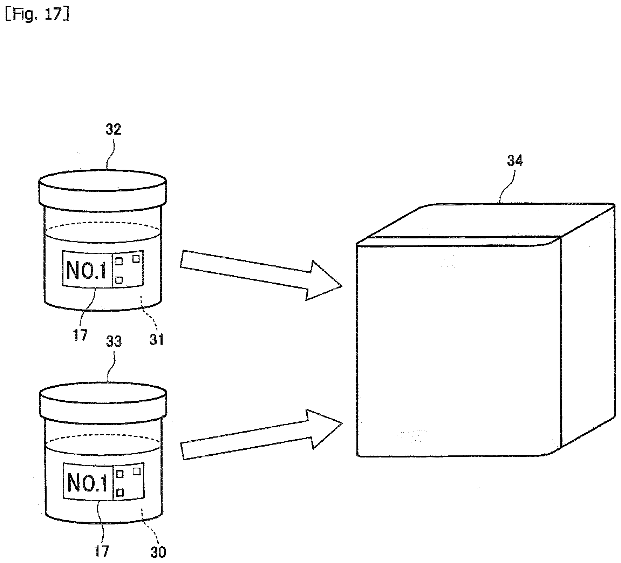

FIG. 16 is a partially enlarged view illustrating another example of a plane shape of a second mesenchymal stem cell 30, and FIG. 17 is a diagram illustrating one example of storing a culture product liquid 31 extracted and a second mesenchymal stem cell 30 stored. FIG. 16 is a magnified view illustrating a plane shape of a second mesenchymal stem cell 30 imaged with an electron microscope 13. According to the observation in the step of third observing a stem cell, the second mesenchymal stem cell 30 (second stem cell) is deformed from the approximately circular (initial plane shape) to the primarily approximately circular and amorphous flat shape, and the step of third observing a stem cell for confirming the fixing of the stem cell 30 to the bottom 27 of the second flat culture container 25 (second culture container) is followed by the step of second culturing a stem cell and the step of fourth observing a stem cell.

In the step of second culturing a stem cell and the step of fourth observing a stem cell e step, the culture liquid 23 fed into the culture container 25 is discharged (discarded) from the culture container 25, and a new culture liquid 23 is fed (stored) into the culture container 25. The culture container 25 is allowed to statically stand (statically without movement) at approximately the same temperature as the body temperature (approx. 36 to 37°C) for 36 to 48 hours, and cultures the second mesenchymal stem cell 30 in the culture container 25 (step of second culturing a stem cell). The total area of the second mesenchymal stem cell 30 fixed to the bottom 27 of the culture container 25, by allowing the culture container 25 to statically stand at an interval of approx. 1 to 2 hours for 36 to 48 hours, in relation to the bottom-surface area of the culture container 25, is observed with the electron microscope 13, and it is determined whether the total area of the stem cell 30 reaches the second target ratio of the bottom-surface area of the culture container 25. The second target ratio of the total area of the second mesenchymal stem cell 30 is 88 to 92% of the bottom-surface area of the culture container 25 (88 to 92% confluent).

Those in charge such as doctors, nurses, and researchers, after confirming the fixing of the second mesenchymal stem cell 30 to the bottom 27 of the culture container 25, click the stem cell third observation end button displayed on the display 16. When the stem cell third observation end button is clicked, the computer 11 displays a step eighth display screen (not shown) on the display 16. The step eighth display screen displays the NO. 1 and the donor data, as well as a stem cell separation end message, a stem cell second collection end message, a stem cell third observation end message, a stem cell fourth observation button, and a culture product liquid first extraction button.

Those in charge click the stem cell fourth observation button on the step eighth display screen, remove the culture container 25 from the specimen holder of the electron microscope 13, and allow the bar code reader 12 to read the QR code of the culture container 25. When the QR code is transmitted from the bar code reader 12 to the computer 11, the computer 11 compares the donor data displayed on the display 16 (donor data read in memory) with the donor data shown by the QR code read by the bar code reader, and when these donor data are matched, the NO. 1 and the donor data are displayed on the display 16, as well as a stem cell separation end message, a stem cell second collection end message, a stem cell third observation end message, a stem cell fourth observation under-way message, and a stem cell fourth observation end button. When these donor data are not matched, the computer 11 displays an error message and a culture cancellation message on the display 16.

Those in charge discharge (discard) the culture liquid 23 fed into the culture container 25 in the step of second culturing a stem cell and the step of fourth observing a stem cell from the culture container 25, and feed (store) a new culture liquid 23 into the culture container 25. The new culture liquid 23 is the same as that fed in a means of first observing a stem cell. Those in charge feed a new culture liquid 23 into the culture container 25, and then set (place) the culture container 25 in the specimen holder of the electron microscope 13. A spacer 39 is positioned between an upper surface 37 of a specimen holder 36 of the electron microscope 13 and a bottom portion 44 of the second flat culture container 25 so as to keep the bottom portion 44 of the culture container 25 raised by the spacer 39, allow the bottom portion 44 of the culture container 25 and the top portion 43 (feed hole 45) of the culture container 25 to stay at higher and lower positions, respectively, and maintain the culture container 25 at a predetermined tilted angle. Also, a spacer 39 may be positioned between the upper surface 37 of the specimen holder 36 of the electron microscope 13 and the top portion 43 of the second flat culture container 25 so as to keep the top portion 43 of the culture container 25 raised by the spacer 39, allow the top portion 43 of the culture container 25 and the bottom portion 44 of the culture container 25 to stay at higher and lower positions, respectively, and maintain the culture container 25 at a predetermined tilted angle. The tilted angle α2 of the second flat culture container 25 in relation to the upper surface 37 of the specimen holder 36 ranges from 2 to 5°, preferably 2 to 3°.

The method for manufacturing a culture product liquid confirms the fixing of the second mesenchymal stem cell 30, then discharges the culture liquid 23 in the culture container 25, and feeds a new culture liquid 23 into the culture container 25, thereby assuredly encouraging the proliferation of the second mesenchymal stem cell 30. The method for manufacturing a culture product liquid places the second flat culture container 25 in relation to an upper surface 37 of the specimen holder 36 at the tilted angle, thereby allowing the second mesenchymal stem cell 30 and the culture liquid 23 in the culture container 25 to tilt to the side of the top portion 43 of the culture container 25 (or the side of the bottom portion 44), causing the water pressure of the second mesenchymal stem cell 30 and the culture liquid 23 to increase on the side of the top portion 43 of the culture container 25 (or the side of the bottom portion 44), allowing the second mesenchymal stem cell 30 to concentrate on the side of the bottom portion 44 of the culture container 25, and thus increasing the activity of the second mesenchymal stem cells 30 to readily and immediately proliferate (differentiate) the second mesenchymal stem cell 30 on the bottom 27 of the culture container 25.

The electron microscope 13 images a magnified view of a plane shape of the second mesenchymal stem cell 30 in the culture container 25 at an interval of approx. 1 to 2 hours, and transmits the magnified view of the plane shape of the imaged stem cell 30 to the computer 11 at an interval of approx. 1 to 2 hours. The computer 11 stores the magnified view of the plane shape of the second mesenchymal stem cell 30 transmitted from the electron microscope 13 and an imaging time in the storage area so as to be associated with the donor identifier (step of fourth storing a stem cell image). The computer 11 displays the magnified view of the plane shape of the second mesenchymal stem cell 30 transmitted from the electron microscope 13 and the imaging time on the display 16.

Those in charge confirm (visually recognize) the magnified view of the plane shape of the second mesenchymal stem cell 30 displayed on the display 16 at an interval of approx. 1 to 2 hours for 36 to 48 hours, observe the total area of the stem cell 30 fixed to the bottom 27 of the culture container 25 in relation to the bottom-surface area of the culture container 25, and determine whether the total area of the stem cell 30 reaches the second target ratio of the bottom-surface area of the culture container 25 (82 to 92% confluent) (a means of fourth observing a stem cell) . Those in charge may directly observe the total area of second mesenchymal stem cell 30 in relation to the bottom-surface area of the culture container 25 from the observation window of the electron microscope 13 at an interval of approx. 1 to 2 hours for 36 to 48 hours, and determine whether the total area of the stem cell 30 reaches the second target ratio of the bottom-surface area of the culture container 25 (88 to 92% confluent).

Those in charge, according to the observation in the step of fourth observing a stem cell, as illustrated in FIG. 13, when the total area of the second mesenchymal stem cell 30 displayed on the display 16 doesn't reach the second target ratio of the bottom-surface area of the culture container 25 (88 to 92% confluent), continuously observe the total area of the stem cell 30 in relation to the bottom-surface area of the culture container 25 at an interval of approx. 1 to 2 hours. When the total area of the second mesenchymal stem cell 30 reaches the second target ratio of all the area of the magnified view displayed on the display 16, it is determined that the total area of the stem cell 30 reaches the second target ratio of the bottom-surface area of the culture container 25.

Those in charge, according to the observation in the step of fourth observing a stem cell, allow the second mesenchymal stem cell 30 to proliferate on the bottom 27 (inner surface of bottom wall) of the second flat culture container 25 (second culture container) to form a colony and to expand its plane shape, and as illustrated in FIG. 14, when the total area of the stem cell 30 displayed on the display 16 reaches the second target ratio of the bottom-surface area of the culture container 25 (88 to 92% confluent), a single type of second mesenchymal stem cell 30 proliferates in the culture container 25, and a culture product liquid 31 containing a predetermined metabolite secreted from a single type of stem cell 30 having activity is manufactured.

The method for manufacturing a culture product liquid gradually reduces the activity of the stem cell 30 when the total area of the second mesenchymal stem cell 30 in relation to the bottom-surface area of the second flat culture container 25 (second culture container) exceeds 92% to proliferate the stem cell 30, and extracts the stem cell 30 from the culture container 25 when the total area of the stem cell 30 in relation to the bottom-surface area of the culture container 25 increases to 88 to 92% for proliferation, thereby maintaining the activity of the stem cell 30, and accordingly proliferating the stem cell 30.

When the total area of the stem cell 30 reaches the second target ratio of the bottom-surface area of the culture container 25, a step of first extracting a culture product liquid for extracting a culture product liquid 31 from the culture container 25 is taken. In the step of first extracting a culture product liquid, the culture product liquid 31 containing a predetermined metabolite secreted from the second mesenchymal stem cell 30 having activity is extracted from the culture container 25. The method for manufacturing a culture product liquid observes the total area at an interval of approx. 1 to 2 hours to properly confirm the total area of the second mesenchymal stem cell 30 in relation to the bottom-surface area of the culture container 25, thereby assuredly confirming that the total area of the stem cell 30 reaches the second target ratio of the bottom-surface area of the culture container 25.

Those in charge such as doctors, nurses, and researchers, after confirming that the total area of the second mesenchymal stem cell 30 reaches the second target ratio of the bottom-surface area of the culture container 25, click the stem cell fourth observation end button displayed on the display 16. When the stem cell fourth observation end button is clicked, the computer 11 displays a step ninth display screen (not shown) on the display 16. The step ninth display screen displays the NO. 1 and the donor data, as well as a stem cell separation end message, a stem cell second collection end message, a stem cell third observation end message, a stem cell fourth observation end message, and a culture product liquid first extraction button.

Those in charge click the culture product liquid first extraction button on the step ninth display screen, and allow the bar code reader 12 to read the QR code of the culture container 25. When the QR code is transmitted from the bar code reader 12 to the computer 11, the computer 11 compares the donor data displayed on the display 16 (donor data read in memory) with the donor data shown by the QR code read by the bar code reader 12, and when these donor data are matched, the NO. 1 and the donor data are displayed on the display 16, as well as a stem cell separation end message, a stem cell second collection end message, a stem cell third observation end message, a stem cell fourth observation end message, and a culture product liquid first extraction end button. When these donor data are not matched, the computer 11 displays an error message and a culture cancellation message on the display 16.

Those in charge, in the step of fourth observing a stem cell, suck from the culture container 25 the culture product liquid 31 converted from the culture liquid 23 fed into the culture container 25, using an injector or a pipette (step of first extracting a culture product liquid), and feed (store) the culture product liquid 31 into the storage container 32. The culture product liquid 31 fed into the storage container 32 is a culture product liquid 31 containing a predetermined metabolite secreted from a single and specific type of mesenchymal stem cell (a single type of stem cell) having activity in which unnecessary mesenchymal stem cell is removed.

Those in charge feed the culture product liquid 31 into the storage container 32, and then affix the first code paper 17 in which a QR code is printed to the outer peripheral surface of the storage container 32. Those in charge click the culture product liquid first extraction end button on the ninth display screen displayed on the display 16, and allow the bar code reader 12 to read the QR code of the storage container 32, and then store in the refrigerator 34 the storage container 32 into which the culture product liquid 31 is fed. The culture product liquid 31 is stored in the refrigerator 34 at approx. 3 to 4°C.

When QR code is transmitted from the bar code reader 12 to the computer 11, the computer 11 compares the donor data displayed on the display 16 (donor data read in memory) with the donor data shown by the QR code read by the bar code reader 12, and when these donor data are matched, the NO. 1 and the donor data are displayed on the display 16, as well as a stem cell separation end message, a stem cell second collection end message, a stem cell third observation end message, a stem cell fourth observation end message, a culture product liquid extraction end message, a culture product liquid manufacturing end button, and a culture product liquid manufacturing continue button. When the manufacturing of a culture product liquid is finished, the culture product liquid manufacturing end button is clicked. The culture product liquid manufacturing end button is clicked to stop the system 10 on the computer 11.

When the culture product liquid manufacturing end button is clicked, a step of collecting a stem cell for collecting a stem cell 30 from the culture container 25 is taken. In the step of collecting a stem cell, the second mesenchymal stem cell 30 proliferated (differentiated) in the culture container 25 is collected from the culture container 25. After the culture container 25 is washed with PBS, a trypsin solution is fed into the culture container 25. When the trypsin solution is fed into the culture container 25, the second mesenchymal stem cell 30 fixed to the bottom 27 of the culture container 25 peels off the bottom 27 by the trypsin solution, floating up to the surface of the trypsin solution. Those in charge suck the second mesenchymal stem cell 30 using an injector or a pipette, and feed (store) the stem cell 30 into the storage container 33 from the injector or the pipette.

The second mesenchymal stem cell 30 fed into the storage container 33 is a single and specific type of mesenchymal stem cell (a single type of stem cell) having activity in which unnecessary mesenchymal stem cell is removed. Those in charge feed the second mesenchymal stem cell 30 (a single type of mesenchymal stem cell) into the storage container 33, then affix the first code paper 17 in which a QR code is printed to the outer peripheral surface of the storage container 33, and store in the refrigerator 34 the storage container 33 into which the second mesenchymal stem cell 30 is fed. The second mesenchymal stem cell 30 (a single type of mesenchymal stem cell) is stored in the refrigerator 34 at approx. 3 to 4°C.

The method for manufacturing a culture product liquid extracts an intermediate-layer bone marrow liquid positioned in an intermediate layer of the bone marrow liquid separated in layers, cultures the intermediate-layer bone marrow liquid together with the culture liquid 23, fixes a first mesenchymal stem cell 26 (first stem cell) to a bottom 27 of the first culture container 24, collects the stem cell 26 from the culture container 24 when the total area of the stem cell 26 reaches the first target ratio of the bottom-surface area of the first culture container 24 while culturing the stem cell 26, collects a second mesenchymal stem cell 30 (second stem cell) positioned in the lower layer (lowermost layer) of the stem cell 26 separated in layers, fixes the stem cell 30 to the bottom 27 of a second culture container 25, feeds a new culture liquid 23 into the culture container 25, cultures the stem cell 30 together with the culture liquid 23, and extracts from the culture container 25 a culture product liquid 31 containing a predetermined metabolite secreted from a single type of stem cell 30 proliferated when the total area of the stem cell 30 reaches a second target ratio of the bottom-surface area of the culture container 25 in the process of culturing the stem cell 30, therefore using a single type of second stem cell 30 having activity cultured, various types of metabolites secreted from various types of stem cells are not contained, but only a predetermined metabolite secreted from a single type of stem cell 30 having activity is contained, and a culture product liquid 31 capable of providing the only specific effect on predetermined diseases, and predetermined cosmetic uses such as skin care, hair care, and body care can be manufactured, a culture product liquid 31 capable of effective use in treating predetermined diseases can be manufactured, and a culture product liquid 31 capable of effective use in predetermined cosmetic uses can be manufactured.

The method for manufacturing a culture product liquid can manufacture a culture product liquid 31, using a pure (clear) second mesenchymal stem cell 30 (second stem cell) obtained by removing unnecessary stem cell, and allow the culture product liquid 31 to contain only a predetermined metabolite secreted from a single type of second stem cell, thereby providing a significant effect of treating predetermined diseases and manufacturing a culture product liquid 31 effective in curing such diseases, which makes it possible to manufacture a culture product liquid 31 having a significant effect on predetermined cosmetic uses. The culture product liquid 31 manufactured by the method for manufacturing a culture product liquid can be used in treating skin diseases such as burns and eczema by skin application, used as eye drops by being instilled in one eye, can be used in treating hay fever by being fed into the nose, and can be used in treating scalp diseases by scalp application. Also, the culture product liquid 31 can be used in treating rough skin, pimples, and suntan, and can be used in cosmetic uses such as skin whitening and wrinkle removal.

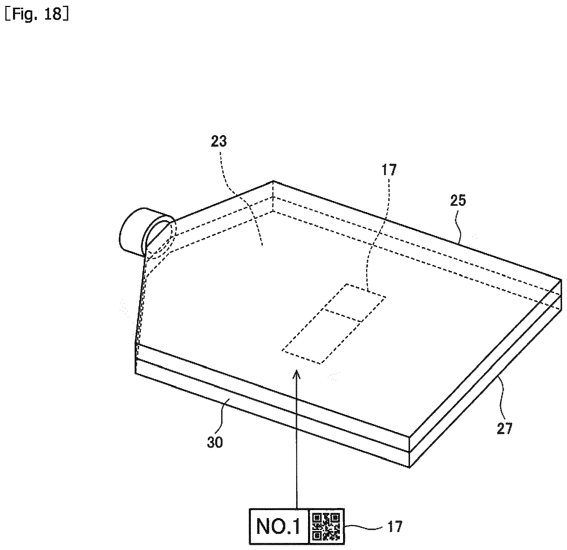

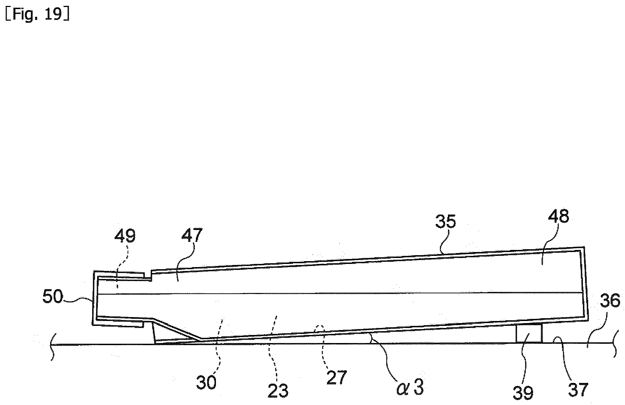

FIG. 18 is an explanatory drawing illustrating one example of a step of fifth observing a stem cell, and FIG. 19 is a side view of the third flat culture container. When the culture product liquid is continuously manufactured, a step of second collecting a stem cell for collecting the second mesenchymal stem cell 30 proliferated (differentiated) in the culture container 25 from the culture container 25 is taken. In the step of second collecting a stem cell, the second mesenchymal stem cell 30 proliferated (differentiated) in the culture container 25 is collected from the culture container 25.

Those in charge such as doctors, nurses, and researchers click the culture product liquid manufacturing continue button displayed on the display 16. When the culture product liquid manufacturing continue button is clicked, the computer 11 displays a step tenth display screen (not shown) on the display 16. The step tenth display screen displays the NO. 1 and the donor data, as well as a stem cell second collection button, a stem cell fifth observation button, a stem cell sixth observation button, and a culture product liquid second extraction button.

Those in charge click the stem cell second collection button on the step tenth display screen, and allow the bar code reader 12 to read the QR code of the culture container 25. When the QR code is transmitted from the bar code reader 12 to the computer 11, the computer 11 compares the donor data displayed on the display 16 (donor data read in memory) with the donor data shown by the QR code read by the bar code reader 12, and when these donor data are matched, the NO. 1 and the donor data are displayed on the display 16, as well as a stem cell second collection under-way message, a stem cell second collection end button, a stem cell fifth observation button, a stem cell sixth observation button, and a culture product liquid second extraction button. When these donor data are not matched, the computer 11 displays an error message and a culture cancellation message on the display 16.

Those in charge, after washing the culture container 25 with PBS, feed a trypsin solution into the culture container 25, collect (suck) the second mesenchymal stem cell 30 floating up to the surface of the trypsin solution using an injector or a pipette, and store the stem cell 26 in the injector or the pipette (a means of second collecting a stem cell) . Those in charge, after collecting the stem cell 30 from the culture container 25, click the stem cell second collection extraction end button. When the stem cell second collection end button is clicked, the computer 11 displays a step eleventh display screen (not shown) on the display 16. The step eleventh display screen displays the NO. 1 and the donor data, as well as a stem cell second collection end message, a stem cell fifth observation button, a stem cell sixth observation button, and a culture product liquid second extraction button.

The step of second collecting a stem cell for collecting the second mesenchymal stem cell 30 from the culture container 25 is followed by a step of fifth observing a stem cell and a step of third fixing a stem cell. In the step of fifth observing a stem cell and the step of third fixing a stem cell, the second mesenchymal stem cell 30 and the culture liquid 23 are fed into the third flat culture container 35 (third culture container), and the culture container 35 is allowed to statically stand (statically without movement) at approximately the same temperature as the body temperature (approx. 36 to 37°C) for 36 to 48 hours.

The deformation of the second mesenchymal stem cell 30 (second stem cell) from the initial plane shape in the culture container 35, by allowing the culture container 35 to statically stand at an interval of approx. 1 to 2 hours for 36 to 48 hours, is observed with the electron microscope 13, and it is determined whether the stem cell 30 is fixed to the bottom 27 of the culture container 35. To the bottom 27 (outer surface of bottom wall) of the culture container 35 into which the second mesenchymal stem cell 30 and the culture liquid 23 are fed is affixed the first code paper 17 in which a QR code for identifying a donor is printed.

Those in charge such as doctors, nurses, and researchers collect the second mesenchymal stem cell 30 from the culture container 25, and then affix the first code paper 17 in which a QR code is printed to the bottom 27 (outer surface of bottom wall) of the third flat culture container 35. Then, those in charge click the stem cell fifth observation button on the step eleventh display screen, and allow the bar code reader 12 to read the QR code of the culture container 25. When the QR code is transmitted from the bar code reader 12 to the computer 11, the computer 11 compares the donor data displayed on the display 16 (donor data read in memory) with the donor data shown by the QR code read by the bar code reader 12, and when these donor data are matched, the NO. 1 and the donor data are displayed on the display 16, as well as a stem cell second collection end message, a stem cell fifth observation under-way message, a stem cell fifth observation end button, a stem cell sixth observation button, and a culture product liquid second extraction button. When these donor data are not matched, the computer 11 displays an error message and a culture cancellation message on the display 16.

Those in charge feed (store) the second mesenchymal stem cell 30 sucked in the injector or the pipette into the culture container 35, and feed (store) the culture liquid 23 in the culture container 35. The third flat culture container 35 (third culture container) is made of transparent glass or transparent plastics, and a flat container whose plane shape is approximately rectangle, having a predetermined capacity and a bottom 27 of a predetermined area, and the area of the bottom 27 is larger than the second flat culture container 25 (second culture container). The third flat culture container 35 includes a top portion 47, a bottom portion 48, and a feed hole 49 provided at the top portion 47. The feed hole 49 is sealed by a cap 50 in watertight manner. The third flat culture container 35 may be a flat container whose plane shape is circular or elliptical, having a predetermined capacity and a bottom 27 of a predetermined area.

The third flat culture container 35 (third culture container) used in the step of fifth observing a stem cell has a capacity of approx. 70 to 80cc (preferably 75cc), and a bottom-surface area of approx. 75 to 108mm². The third flat culture container has a side of approx. 8.7 to 10.4mm long. The culture liquid 23 is the same as that fed in the step of first observing a stem cell. The second mesenchymal stem cell 30 fed into the culture container 35 is fixed to the bottom 27 of the culture container 35 as time elapses, cultured by the culture liquid 23, and gradually proliferated (differentiated) on the bottom 27 of the culture container 35 to form a colony.

Those in charge, after feeding the second mesenchymal stem cell 30 and the culture liquid 23 into the culture container 35, set (place) the culture container 35 in a specimen holder of the electron microscope 13. A spacer 39 is positioned between an upper surface 37 of a specimen holder 36 of the electron microscope 13 and a bottom portion 48 of the third flat culture container 35 so as to keep the bottom portion 48 of the culture container 35 raised by the spacer 39, allow the bottom portion 48 of the culture container 25 and the top portion 47 of the culture container 25 (feed hole 49) to stay at higher and lower positions, respectively, and maintain the culture container 35 maintained at a predetermined tilted angle. Also, a spacer 39 is positioned between the upper surface 37 of the specimen holder 36 of the electron microscope 13 and the top portion 48 of the third flat culture container 35 so as to keep the top portion 47 of the culture container 35 by the spacer 39, allow the top portion 47 of the culture container 35 and the bottom portion 49 of the culture container 35 to stay at higher and lower positions, respectively, and maintain the culture container 35 maintained at a predetermined tilted angle. The tilted angle α2 of the third flat culture container 35 in relation to the upper surface 37 of the specimen holder 36 ranges from 2 to 5°, preferably 2 to 3°.

The method for manufacturing a culture product liquid places the third flat culture container 35 in relation to the upper surface 37 of the specimen holder 36 at the tilted angle, thereby allowing the second mesenchymal stem cell 30 and the culture liquid 23 in the culture container 35 to tilt to the side of the top portion 47 of the culture container 35 (or the side of the bottom portion 48), causing the water pressure of the second mesenchymal stem cell 30 and the culture liquid 23 to increase on the side of the top portion 47 of the culture container 35 (or the side of the bottom portion 48), allowing the second mesenchymal stem cell 30 to concentrate on the side of the bottom portion 48 of the culture container 35, and thus increasing the activity of the second mesenchymal stem cells 30 to readily and immediately fix the second mesenchymal stem cell 30 on the bottom 27 of the culture container 35.

The electron microscope 13 images a magnified view of a plane shape of the second mesenchymal stem cell 30 fed into the culture container 35 at an interval of approx. 1 to 2 hours, and transmits the magnified view of the plane shape of the imaged stem cell 30 to the computer 11 at an interval of approx. 1 to 2 hours. The computer 11 stores a magnified view of a plane shape of the second mesenchymal stem cell 30 transmitted from the electron microscope 13 and an imaging time in the storage area so as to be associated with the donor identifier (step of fifth storing a stem cell image). The computer 11 displays the magnified view of the plane shape of the stem cell 30 transmitted from the electron microscope 13 and the imaging time on the display 16. Those in charge confirm (visually recognize) the magnified view of the plane shape of the stem cell 30 displayed on the display 16 at an interval of approx. 1 to 2 hours for 36 to 48 hours, and observe changes in the plane shape of the stem cell 30 (step of fifth observing a stem cell) . Those in charge may directly observe changes in the plane shape of the stem cell 30 from the observation window of the electron microscope 13 at an interval of approx. 1 to 2 hours for 36 to 48 hours.

The initial plane shape of the second mesenchymal stem cell 30 is an approximately circular shape, and when the plane shape of the stem cell 30 is the approximately circular shape, the stem cell 30 is not fixed to the bottom 27 of the culture container 35 (inner surface of bottom wall), which doesn't allow the stem cell 30 to start proliferation (differentiation). The plane shape of the second mesenchymal stem cell 30 deformed is primarily the approximately circular shape before fixing, and a flat shape when the stem cell 30 elongates in amorphous form in one direction, thereby fixing the stem cell 30 to the bottom 27 of the culture container 35 (inner surface of bottom wall) and allowing the stem cell 30 to start proliferation (differentiation).

Those in charge, according to the observation in the step of fifth observing a stem cell, when the plane shape of the second mesenchymal stem cell 30 displayed on the display 16 remains observed as an approximately circular shape (see FIG. 12 cited by reference), determine that the stem cell 30 is not fixed to the bottom 27 of the culture container 35 (inner surface of bottom wall), and continuously observe changes in the plane shape of the stem cell 30 at an interval of approx. 1 to 2 hours. Those in charge, according to the observation in a means of third observing a stem cell, when the plane shape of the second mesenchymal stem cell 30 displayed on the display 16 is deformed from the approximately circular to the primarily approximately circular and amorphous flat shape (see FIG. 13 cited by reference), determine that the stem cell 30 is fixed to the bottom 27 of the culture container 35 (step of third fixing a stem cell).

The use of a large culture container in which the capacity exceeds 80cc to fix the second mesenchymal stem cell 30 and the bottom-surface area exceeds 108mm² fails to readily fix the stem cell 30 to the bottom surface of the container and slows down the proliferation of the stem cell 30, but the method for manufacturing a culture product liquid, using the third flat culture container 35 having the capacity and the bottom-surface area, can readily fix the stem cell 30 to the bottom 27 of the culture container 35 and immediately be proliferate the stem cell 30 in the culture container 35.

The method for manufacturing a culture product liquid allows the culture container 35 to statically stand at approximately the same temperature as the body temperature for 36 to 48 hours, and observes the deformation of the second mesenchymal stem cell 30 from the initial plane shape in the culture container 35 at an interval of approx. 1 to 2 hours for 36 to 48 hours, thereby never failing to confirm the deformation of the stem cell 30 and properly confirming the fixing of the stem cell 30 to the bottom 27 of the culture container 35.

According to the observation in the step of fifth observing a stem cell, the second mesenchymal stem cell 30 (second stem cell) is deformed from the approximately circular (initial plane shape) to the primarily approximately circular and amorphous flat shape, and the step of third fixing a stem cell for confirming the fixing of the stem cell 30 to the bottom 27 of the third flat culture container 35 (third culture container) is followed by the step of third culturing a stem cell and a step of sixth observing a stem cell.

In the third culturing a stem cell and the step of sixth observing a stem cell, the culture liquid 23 fed into the culture container 35 is discharged (discarded) from the culture container 35, and a new culture liquid 23 is fed (stored) into the culture container 35. The culture container 35 is allowed to statically stand (statically without movement) at approximately the same temperature as the body temperature (approx. 36 to 37°C) for 36 to 48 hours, and cultures the second mesenchymal stem cell 30 in the culture container 35 (step of third culturing a stem cell) . The total area of the second mesenchymal stem cell 30 fixed to the bottom 27 of the culture container 35, by allowing the culture container 35 to statically stand at an interval of approx. 1 to 2 hours for 36 to 48 hours, in relation to the bottom-surface area of the culture container 35, is observed with the electron microscope 13, and it is determined whether the total area of the stem cell 30 reaches the third target ratio of the bottom-surface area of the culture container 35. The third target ratio of the total area of the second mesenchymal stem cell 30 is 88 to 92% of the bottom-surface area of the culture container 35 (88 to 92% confluent).

Those in charge such as doctors, nurses, and researchers, after confirming the fixing of the second mesenchymal stem cell 30 to the bottom 27 of the culture container 35, click the stem cell fifth observation end button displayed on the display 16. When the stem cell fifth observation end button is clicked, the computer 11 displays a step twelfth display screen (not shown) on the display 16. The step twelfth display screen displays the NO. 1 and the donor data, as well as a stem cell second collection end message, a stem cell fifth observation end message, a stem cell sixth observation button, and a culture product liquid second extraction button.

Those in charge click the stem cell sixth observation button on the step twelfth display screen, remove the culture container 35 from the specimen holder of the electron microscope 13, and allow the bar code reader 12 to read the QR code of the culture container 35. When the QR code is transmitted from the bar code reader 12 to the computer 11, the computer 11 compares the donor data displayed on the display 16 (donor data read in memory) with the donor data shown by the QR code read by the bar code reader, and when these donor data are matched, the NO. 1 and the donor data are displayed on the display 16, as well as a stem cell second collection end message, a stem cell fifth observation end message, a stem cell sixth observation under-way message, a stem cell sixth observation end button, and a culture product liquid second extraction button. When these donor data are not matched, the computer 11 displays an error message and a culture cancellation message on the display 16.

Those in charge discharge (discard) the culture liquid 23 fed into the culture container 35 in the step of fifth observing a stem cell from the culture container 35, and feed (store) a new culture liquid 23 into the culture container 35. The new culture liquid 23 is the same as that fed in a means of first observing a stem cell. Those in charge feed a new culture liquid 23 into the culture container 35, and then set (place) the culture container 35 in the specimen holder 36 of the electron microscope 13.

A spacer 39 is positioned between an upper surface 37 of a specimen holder 36 of the electron microscope 13 and a bottom portion 48 of the third flat culture container 35 so as to keep the bottom portion 48 of the culture container 35 raised by the spacer 39, allow the bottom portion 48 of the culture container 25 and the top portion 47 of the culture container 25 (feed hole 49) to stay at higher and lower positions, respectively, and maintain the culture container 35 at a predetermined tilted angle. Also, a spacer 39 may be positioned between the upper surface 37 of the specimen holder 36 of the electron microscope 13 and the top portion 48 of the third flat culture container 35 so as to keep the top portion 47 of the culture container 35 raised by the spacer 39, allow the top portion 47 of the culture container 35 and the bottom portion 49 of the culture container 35 to stay at higher and lower positions, respectively, and maintain the culture container 35 at a predetermined tilted angle. The tilted angle α2 of the third flat culture container 35 in relation to the upper surface 37 of the specimen holder 36 ranges from 2 to 5°, preferably 2 to 3°.

The method for manufacturing a culture product liquid confirms the fixing of the second mesenchymal stem cell 30, then discharges the culture liquid 23 in the culture container 35, and feeds a new culture liquid 23 into the culture container 35, thereby assuredly encouraging the proliferation of the second mesenchymal stem cell 30. The method for manufacturing a culture product liquid places the third flat culture container 35 in relation to an upper surface 37 of the specimen holder 36 at the tilted angle, thereby allowing the second mesenchymal stem cell 30 and the culture liquid 23 in the culture container 35 to tilt to the side of the top portion 47 of the culture container 35 (or the side of the bottom portion 48), causing the water pressure of the second mesenchymal stem cell 30 and the culture liquid 23 to increase on the side of the top portion 47 of the culture container 35 (or side of the bottom portion 48), allowing the second mesenchymal stem cell 30 to concentrate on the side of the bottom portion 48 of the culture container 35, and thus increasing the activity of the second mesenchymal stem cells 30 to readily and immediately proliferate (differentiate) the second mesenchymal stem cell 30 on the bottom 27 of the culture container 35.

The electron microscope 13 images a magnified view of a plane shape of the second mesenchymal stem cell 30 in the culture container 35 at an interval of approx. 1 to 2 hours, and transmits the magnified view of the plane shape of the imaged stem cell 30 to the computer 11 at an interval of approx. 1 to 2 hours. The computer 11 stores the magnified view of the plane shape of the second mesenchymal stem cell 30 transmitted from the electron microscope 13 and an imaging time in the storage area so as to be associated with the donor identifier (step of sixth storing a stem cell image) . The computer 11 displays the magnified view of the plane shape of the second mesenchymal stem cell 30 transmitted from the electron microscope 13 and the imaging time on the display 16.

Those in charge confirm (visually recognize) the magnified view of the plane shape of the second mesenchymal stem cell 30 displayed on the display 16 at an interval of approx. 1 to 2 hours for 36 to 48 hours, observe the total area of the stem cell 30 fixed to the bottom 27 of the culture container 35 in relation to the bottom-surface area of the culture container 35, and determine whether the total area of the stem cell 30 reaches the third target ratio of the bottom-surface area of the culture container 35 (82 to 92% confluent) (step of sixth observing a stem cell) . Those in charge may directly observe the total area of the second mesenchymal stem cell 30 in relation to the bottom-surface area of the culture container 35 from the observation window of the electron microscope 13 at an interval of approx. 1 to 2 hours for 36 to 48 hours, and determine whether the total area of the stem cell 30 reaches the third target ratio of the bottom-surface area of the culture container 35 (88 to 92% confluent).

Those in charge, according to the observation in the step of sixth observing a stem cell, when the total area of the second mesenchymal stem cell 30 displayed on the display 16 doesn't reach the third target ratio of the bottom-surface area of the culture container 35 (88 to 92% confluent), continuously observe the total area of the stem cell 30 in relation to the bottom-surface area of the culture container 35 at an interval of approx. 1 to 2 hours (see FIG. 13 cited by reference) . When the total area of the second mesenchymal stem cell 30 reaches the third target ratio of all the area of the magnified view displayed on the display 16, it is determined that the total area of the stem cell 30 reaches the third target ratio of the bottom-surface area of the culture container 35.

According to the observation in the step of sixth observing a stem cell, the second mesenchymal stem cell 30 proliferates on the bottom 27 (inner surface of bottom wall) of the third flat culture container 35 (third culture container) to form a colony and expands its plane shape, and when the total area of the stem cell 30 displayed on the display 16 reaches the third target ratio of the bottom-surface area of the culture container 35 (88 to 92% confluent) (see FIG. 14 cited by reference), a single type of second mesenchymal stem cell 30 proliferates in the culture container 35, and a culture product liquid 31 containing a predetermined metabolite secreted from a single type of stem cell 30 having activity is manufactured.

The method for manufacturing a culture product liquid gradually reduces the activity of the stem cell 30 when the total area of the second mesenchymal stem cell 30 in relation to the bottom-surface area of the third flat culture container 35 (third culture container) exceeds 92% to proliferate the stem cell 30, and extracts the stem cell 30 from the culture container 35 when the total area of the stem cell 30 in relation to the bottom-surface area of the culture container 35 increases to 88 to 92% for proliferation, thereby maintaining the activity of the stem cell 30, and accordingly proliferating the stem cell 30.

When the total area of the stem cell 30 reaches the third target ratio of the bottom-surface area of the culture container 35, a step of second extracting a culture product liquid for extracting the culture product liquid 31 from the culture container 35 is taken. In the step of second extracting a culture product liquid, the culture product liquid 31 containing a predetermined metabolite secreted from the second mesenchymal stem cell 30 having activity is extracted from the culture container 35. The method for manufacturing a culture product liquid observes the total area at an interval of approx. 1 to 2 hours to properly confirm the total area of the second mesenchymal stem cell 30 in relation to the bottom-surface area of the culture container 35, thereby assuredly confirming that the total area of the stem cell 30 reaches the third target ratio of the bottom-surface area of the culture container 35.

Those in charge such as doctors, nurses, and researchers, after confirming that the total area of the second mesenchymal stem cell 30 reaches the third target ratio of the bottom-surface area of the culture container 35, click the stem cell sixth observation end button displayed on the display 16. When the stem cell sixth observation end button is clicked, the computer 11 displays a step thirteenth display screen (not shown) on the display 16. The step thirteenth display screen displays the NO. 1 and the donor data, as well as a stem cell second collection end message, a stem cell fifth observation end message, a stem cell sixth observation end message, and a culture product liquid second extraction button.

Those in charge, in the step of sixth observing a stem cell, suck from the culture container 35 the culture product liquid 31 converted from the culture liquid 23 fed into the culture container 35, using an injector or a pipette (step of second extracting a culture product liquid), and feed (store) the culture product liquid 31 into the storage container 32. The culture product liquid 31 fed into the storage container 32 is a culture product liquid 31 containing a predetermined metabolite secreted from a single and specific type of mesenchymal stem cell (a single type of stem cell) having activity in which unnecessary mesenchymal stem cell is removed.

Those in charge feed the culture product liquid 31 into the storage container 32, and then affix the first code paper 17 in which a QR code is printed to the outer peripheral surface of the storage container 32. Those in charge click the culture product liquid second extraction end button on the thirteenth display screen displayed on the display 16, allow the bar code reader 12 to read the QR code of the storage container 32, and then store in the refrigerator 34 the storage container 32 into which the culture product liquid 31 is fed. The culture product liquid 31 is stored in the refrigerator 34 at approx. 3 to 4°C.

When the QR code is transmitted from the bar code reader 12 to the computer 11, the computer 11 compares the donor data displayed on the display 16 (donor data read in memory) with the donor data shown by the QR code read by the bar code reader 12, and when these donor data are matched, the NO. 1 and the donor data are displayed on the display 16, as well as a culture product liquid extraction end message and a culture product liquid manufacturing end button. Those in charge click the culture product liquid manufacturing end button. The culture product liquid manufacturing end button is clicked to stop the system 10 on the computer 11.

When the culture product liquid manufacturing end button is clicked, a step of collecting a stem cell for collecting the stem cell 30 from the culture container 25 is taken. In the step of collecting a stem cell, the second mesenchymal stem cell 30 proliferated (differentiated) in the culture container 35 is collected from the culture container 35. After washing the culture container 35 with PBS, those in charge feed a trypsin solution into the culture container 35, suck the second mesenchymal stem cell 30 floating up to the surface of the trypsin solution using an injector or a pipette, and feed (store) the stem cell 30 into the storage container 33 from the injector or the pipette.

The second mesenchymal stem cell 30 fed into the storage container 33 is a single and specific type of mesenchymal stem cell (a single type of stem cell) having activity in which unnecessary mesenchymal stem cell is removed. Those in charge feed the second mesenchymal stem cell 30 (a single type of mesenchymal stem cell) into the storage container 33, then affix the first code paper 17 in which a QR code is printed to the outer peripheral surface of the storage container 33, and store in the refrigerator 34 the storage container 33 into which the second mesenchymal stem cell 30 is fed. The second mesenchymal stem cell 30 (a single type of mesenchymal stem cell) is stored in the refrigerator 34 at approx. 3 to 4°C.

The method for manufacturing a culture product liquid, which collects the second stem cell 30 from the second culture container 25 after extracting a culture product liquid 31 from the second culture container 25, cultures the stem cell 30 together with a culture liquid 23, fixes the stem cell 30 to the bottom 27 of a third culture container 35 having a larger capacity than the second culture container 25, feeds a new culture liquid 23 into the culture container 35, cultures the stem cell 30, and extracts from the culture container 35 the culture product liquid 31 containing a predetermined metabolite secreted from a single type of second stem cell 30 having activity proliferated when the total area of the stem cell 30 reaches the third target ratio of the bottom-surface area of the culture container 35 in the process of culturing the stem cell 30, therefore in the process of culturing the stem cell 30, various types of stem cells are not cultured, various types of metabolites secreted from various types of stem cells are not contained, only a predetermined metabolite secreted from a single type of second stem cell 30 having activity is contained, a culture product liquid 31 providing the only specific effect on predetermined diseases and predetermined cosmetic uses can be manufactured, and a culture product liquid 31 capable of effective use in treating predetermined diseases and a culture product liquid 31 capable of effective use in predetermined cosmetic uses can be manufactured.

### EXPLANATIONS OF LETTERS OR NUMERALS

10 Culture product liquid manufacturing system
11 Computer
12 Bar code reader
13 Electron microscope
14 Keyboard
15 Mouse
16 Display
17 First code paper
18 Raw bone marrow liquid
19 Glass test tube
20 Test tube rack
21 Constant temperature bath
22 Intermediate-layer bone marrow liquid
23 Culture liquid
24 First culture container (first flat culture container)
25 Second culture container (second flat culture container)
26 First mesenchymal stem cell (first stem cell)
27 Bottom
28 Centrifugal separator
29 Glass test tube
30 Second mesenchymal stem cell (second stem cell)
31 Culture product liquid
32 Storage container
33 Storage container
34 Refrigerator
35 Third culture container (third flat culture container)
36 Specimen holder
37 Upper surface
38 Bottom portion
39 Spacer
40 Top portion
41 Feed hole
42 Cap
43 Top portion
44 Bottom portion
45 Feed hole
46 Cap
47 Top portion
48 Bottom portion
49 Feed hole
50 Cap

## Claims

1. A method for manufacturing a culture product liquid, using a stem cell made from a bone marrow liquid collected from a donor, the method comprising the steps of:
separating a bone marrow liquid for separating in layers a bone marrow liquid collected from a donor;
extracting a bone marrow liquid for extracting an intermediate-layer bone marrow liquid positioned in an intermediate layer of the bone marrow liquid separated in layers by the step of separating a bone marrow liquid;
first fixing a stem cell for feeding the intermediate-layer bone marrow liquid extracted by the step of extracting a bone marrow liquid and a predetermined culture liquid into a first culture container having a predetermined capacity and a bottom surface of a predetermined area, allowing the first culture container to statically stand for a predetermined period of time, and fixing a first stem cell contained in the intermediate-layer bone marrow liquid to a bottom surface of the first culture container;
first culturing a stem cell for discharging the culture liquid in the first culture container after fixing the first stem cell to the bottom surface of the first culture container by the step of first fixing a stem cell, feeding a new culture liquid into the first culture container, allowing the first culture container to statically stand for a predetermined period of time, and culturing the first stem cell until the total area of the first stem cell reaches a first target ratio of the bottom-surface area of the first culture container area;
first collecting a stem cell for collecting the first stem cell from the first culture container when the total area of the first stem cell reaches the first target ratio in the process of culturing the first stem cell by the step of first culturing a stem cell;
separating a stem cell for centrifugally separating in layers the first stem cell collected by the step of first collecting a stem cell;
second collecting a stem cell for collecting a second stem cell positioned in the lowermost layer of the first stem cell separated in layers by the step of separating a stem cell;
second fixing a stem cell for feeding the second stem cell collected by the step of second collecting a stem cell and a predetermined culture liquid into a second culture container having a larger capacity and a bottom surface of a larger bottom-surface area than the first culture container, allowing the second culture container to statically stand for a predetermined period of time, and fixing the second stem cell to a bottom surface of the second culture container;
second culturing a stem cell for discharging the culture liquid in the second culture container after fixing the second stem cell to the bottom surface of the second culture container by the step of second fixing a stem cell, feeding a new culture liquid into the second culture container, allowing the second culture container to statically stand for a predetermined period of time, and culturing the second stem cell until the total area of the second stem cell reaches a second target ratio of the bottom-surface area of the second culture container; and
first extracting a culture product liquid for extracting from the second culture container a culture product liquid containing a predetermined metabolite secreted from the single type of second stem cell proliferated when the total area of the second stem cell reaches the second target ratio in the process of culturing in the step of second culturing a stem cell.

2. The method for manufacturing a culture product liquid according to claim 1, wherein in the steps of first fixing a stem cell and first culturing a stem cell, the first culture container is allowed to statically stand at a predetermined tilted angle for a predetermined period of time, and in the steps of second fixing a stem cell and second culturing a stem cell, the second culture container is allowed to statically stand at a predetermined tilted angle for a predetermined period of time.

3. The method for manufacturing a culture product liquid according to claim 1 or 2, comprising the steps of:
second collecting a stem cell for collecting the second stem cell from the second culture container after extracting a culture product liquid from the second culture container by the step of first extracting a culture product liquid;
third fixing a stem cell for feeding the second stem cell collected by the step of second collecting a stem cell and a new culture liquid into a third culture container having a predetermined capacity and a bottom surface of a predetermined area, the capacity and the bottom-surface area being larger than the second culture container, allowing the third culture container to statically stand for a predetermined period of time, and fixing the second stem cell to a bottom surface of the third culture container;
third culturing a stem cell for discharging the culture liquid in the third culture container after fixing the second stem cell to the bottom surface of the third culture container by the step of third fixing a stem cell, feeding a new culture liquid into the third culture container, allowing the third culture container to statically stand for a predetermined period of time, and culturing the second stem cell until the total area of the second stem cell reaches a third target ratio of the bottom-surface area of the third culture container; and
second extracting a culture product liquid for extracting from the third culture container a culture product liquid containing a predetermined metabolite secreted from the single type of second stem cell proliferated when the total area of the second stem cell reaches the third target ratio in the process of culturing in the step of third culturing a stem cell.

4. The method for manufacturing a culture product liquid according to claim 3, wherein in the steps of third fixing a stem cell and third culturing a stem cell, the third culture container is allowed to statically stand at a predetermined tilted angle for a predetermined period of time.

5. The method for manufacturing a culture product liquid according to any one of claims 1 to 4, wherein in the step of separating a bone marrow liquid, 2 to 3cc of a bone marrow liquid is collected from the donor, 2 to 3cc of the bone marrow liquid is fed into a vertically elongating separation container, the separation container is allowed to statically stand at approximately the same temperature as the body temperature for a predetermined period of time, and the bone marrow liquid is vertically separated in layers in the separation container, and in the step of extracting a bone marrow liquid, the intermediate-layer bone marrow liquid positioned in the intermediate layer of the bone marrow liquid separated in layers in the separation container is extracted.

6. The method for manufacturing a culture product liquid according to any one of claims 1 to 5, wherein the capacity of the first culture container is approx. 20 to 30cc, the initial plane shape of the first stem cell is an approximately circular shape, and the plane shape of the first stem cell deformed is primarily the approximately circular shape, and a flat shape when the first stem cell elongates in amorphous form in one direction, and in the step of first fixing a stem cell, the first culture container is allowed to statically stand at approximately the same temperature as the body temperature for a period of 12 to 24 hours, the deformation of the first stem cell from the initial plane shape in the first culture container is observed at an interval of approx. 1 to 2 hours for the period of 12 to 24 hours, and when the first stem cell is deformed from the initial plane shape to the amorphous flat shape, it is determined that the first stem cell is fixed to the bottom surface of the first culture container.

7. The method for manufacturing a culture product liquid according to any one of claims 1 to 6, wherein in the step of first collecting a stem cell, the first culture container is allowed to statically stand at approximately the same temperature as the body temperature for a period of 36 to 48 hours, and the total area of the first stem cell fixed to the bottom surface of the first culture container in relation to the bottom-surface area of the first culture container area is observed at an interval of approx. 1 to 2 hours for the period of 36 to 48 hours.

8. The method for manufacturing a culture product liquid according to claim 7, wherein the first target ratio of the total area of the first stem cell is 70 to 80% of the bottom-surface area of the first culture container area.

9. The method for manufacturing a culture product liquid according to any one of claims 1 to 8, wherein in the step of separating a stem cell, the first stem cell is fed into a separation container, the separation container is placed in a centrifugal separator to centrifugally separate the first stem cell, and in the step of first collecting a stem cell, the second stem cell positioned in the lowermost layer of the first stem cell centrifugally separated in layers in the separation container is collected.

10. The method for manufacturing a culture product liquid according to any one of claims 3 to 9, wherein the capacity of the second culture container is approx. 40 to 60cc, the capacity of the third culture container is approx. 70 to 80cc, the initial plane shape of the second stem cell is an approximately circular shape, the plane shape of the second stem cell deformed is primarily the approximately circular shape, and a flat shape when the second stem cell elongates in amorphous form in one direction, and in the steps of second fixing a stem cell and third fixing a stem cell, the second and the third culture containers are allowed to statically stand at approximately the same temperature as the body temperature for a period of 36 to 48 hours, the deformation of the second stem cell from the initial plane shape in the second and the third culture containers is observed at an interval of approx. 1 to 2 hours for the period of 36 to 48 hours, and when the second stem cell is deformed from the initial plane shape to the amorphous flat shape, it is determined that the second stem cell is fixed to the bottom surfaces of the second and the third culture containers.

11. The method for manufacturing a culture product liquid according to any one of claims 3 to 10, wherein in the steps of second collecting a stem cell and third collecting a stem cell, the second and the third culture containers are allowed to statically stand at approximately the same temperature as the body temperature for a period of 36 to 48 hours, the total area of the second stem cell fixed to the bottom surface of the second and the third culture containers in relation to the bottom-surface area of the second and the third culture containers is observed at an interval of approx. 1 to 2 hours for the period of 36 to 48 hours.

12. The method for manufacturing a culture product liquid according to any one of claims 3 to 11, wherein the second target ratio of the total area of the second stem cell is 88 to 92% of the bottom-surface area of the second culture container, and the third target ratio of the total area of the second stem cell is 88 to 92% of the bottom-surface area of the third culture container.

13. The method for manufacturing a culture product liquid according to any one of claims 1 to 12, wherein the first and second stem cells are mesenchymal stem cells.
